# EUROPEAN PATENT APPLICATION

(11) **EP 4 235 177 A1**
(43) Date of publication of application: **30.08.2023**
(21) Application number: 21882929.9
(22) Date of filing: 22.10.2021
(51) Int. Cl.: G01N 33/543, G01N 21/64

(54) **TARGET SUBSTANCE DETECTION METHOD, DEVICE, AND REAGENT**

(30) Priority: 23.10.2020 JP 2020178485
(71) Applicant: Tauns Laboratories, Inc., Shizuoka 4102325 (JP)
(72) Inventor: TAKEUCHI Rikiya, Sunto-gun Shizuoka 411-0903 (JP); OTA Toshiya, Sunto-gun Shizuoka 411-0903 (JP); SHIRAKI Saaya, Sunto-gun Shizuoka 411-0903 (JP); MORI Ayami, Sunto-gun Shizuoka 411-0903 (JP); OHTA Hirokazu, Sunto-gun Shizuoka 411-0903 (JP)
(74) Representative: Eidelsberg, Olivier Nathan
(86) International application number: PCT/JP2021/039132
(87) International publication number: WO 2022/085790

(57) **Abstract**

Provided is a target substance detection method that includes: forming a complex in a first reaction field, by sandwiching a target substance between a first trapping substance immobilized on a solid phase and a second trapping substance labeled with a labeling substance; separating a moiety that includes the labeling substance from the complex; moving the moiety to a second reaction field; and detecting the target substance by a signal based on the labeling substance in the second reaction field. As a result of this technology, a target substance in a sample can be detected with greater sensitivity and at a lower cost than when using conventional technology.

## Description

### Technical Field

The present invention relates to a detection method and a detection device for detecting a target substance in a sample. Particularly, the present invention relates to a detection method, a device and a reagent capable of detecting a target substance trapped by an immune reaction with a great sensitivity and at a low cost.

### Background Art

An immunoassay has heretofore been known which discovers a disease or quantitatively analyzes therapeutic effects or the like by detecting a particular antigen or antibody associated with the disease as a biomarker. In recent years, there has been a growing demand for highly sensitive detection to detect a trace amount of a biomarker for the purpose of detecting a more effective biomarker. An enzyme-linked immunosorbent assay (also referred to as "ELISA" in the present specification), one of such immunoassay techniques, is a method of detecting a target antigen, antibody or nucleic acid contained in a sample solution through the use of enzyme reaction while trapping the target with a specific antibody, an antigen that binds to the target antibody, or a complementary nucleic acid, and is widely prevalent because of its advantages such as a cost.

Although an ELISA is an assay capable of quantitatively detecting a target substance, most of the currently prevalent ELISA techniques are operated on a 96-well microplate, have a large number of steps, and require a complicated operation. Furthermore, such an ELISA undergoes a plurality of reaction steps and therefore requires a great deal of labor and time for obtaining measurement results. Hence, it is desired to shorten an operating time or a reaction time. Moreover, more highly sensitive detection is demanded.

It is considered that a highly sensitive detection can be achieved by improving the reactivity between an enzyme and a substrate. For this purpose, a technique of liberating an immune complex (labeled compound) from a solid phase and reacting the immune complex in a free liquid medium has been proposed (Patent Literature 1). In this Patent Literature 1, components of the immune complex are designed such that a biotin or a functionalized azo dye and an avidin are introduced in the immune complex. For example, an antibody bound to a reporter group via streptavidin-biotin binding is prepared as a labeled antibody. When the immune complex is formed on a solid phase, the reporter group is introduced into the immune complex via streptavidin-biotin binding. Then, an excess of streptavidin is added thereto so that streptavidin in the complex is replaced with the added streptavidin to liberate the reporter molecule from the immune complex. Hence, in this technique, the reporter molecule is detected in a free liquid medium and can thereby be associated with an analyte concentration in a specimen. It is considered that the detection of the reporter molecule that has been liberated is more advantageous than that of the same bound to the immune complex immobilized on a solid phase, in terms of reactivity with an analysis reagent.

Meanwhile, a microreactor where reaction is performed in a micro container of a µm scale has been developed by microfabrication technology. If a microreactor having a micro space serving as a reaction field is used, it can be expected that a reaction rate is improved. The realization of an experimental operation such as extraction or separation on a microreactor using a micro channel or reaction chamber is often called "lab on a chip". It has been proposed that a biological material is isolated by forming a microreactor in a disc having a flat circular shape and dispensing a sample solution by centrifugal force caused by rotation (Patent Literature 2). It has also been proposed that immunoassay procedures, which have been performed in wells, are implemented in a microreactor by forming a plurality of chambers, channels, reservoirs and the like in a disc having a flat circular shape and carrying out the liquid feed of a reagent and waste liquid disposal by rotation of the disc (Patent Literature 3).

Further, a method using a labeling bead in an immunoassay in a microreactor has been proposed for the purpose of detecting a target substance having a low concentration (Patent Literature 4). For example, Patent Literature 4 discloses a disc having a flat circular shape, which is provided with a bead filled portion which is filled with a labeling bead of which surface is modified with an antibody, as well as a detection region. An antigen in a sample solution injected to the disc is trapped by the antibody on the labeling bead, and a labeling bead complex bound by the antigen on the labeling bead is sent to the detection region by the rotation of the disc and trapped by an antibody immobilized on a detection area. The quantity of the bound labeling bead complexes is measured with an optical reading means of an optical disc device to detect a target substance in the sample, as disclosed therein.

In recent years, a system for carrying out an ELISA by a combined approach of beads and an array provided with femtoliter-sized microwells has been developed. Specifically, an antigen in a sample solution is subjected to formation of a sandwich ELISA (immune complex of an antibody, an antigen, and an enzyme-labeled antibody) on a bead. Subsequently, a solution containing the beads is introduced, together with a substrate, onto an array provided with femtoliter-sized microwells. Some of the introduced beads are housed in the microwells by gravitational sedimentation. Then, unhoused beads are removed, and the array is imaged. The microwells are designed such that only one bead is placed per well. The substrate only in a well where a bead that has formed an immune complex is housed is converted to a fluorescent substance by the labeling enzyme. The detection of a target substance can be carried out by the digital counting of the fluorescent well even if the target substance has a low concentration (Non Patent Literatures 1 and 2).

### Citation List

### Patent Literature

Patent Literature 1
   Japanese Patent Laid-Open (kohyo) No. H06-505802
Patent Literature 2
   Japanese Patent Laid-Open (kokai) No. 2008-185423
Patent Literature 3
   Japanese Patent Laid-Open (kohyo) No. 2002-503331
Patent Literature 4
   Japanese Patent Laid-Open (kokai) No. 2012-255772 Non Patent Literature

Non Patent Literature 1
   David H. Wilson et al., The Simoa HD-1 Analyzer: A Novel Fully Automated Digital Immunoassay Analyzer with Single-Molecule Sensitivity and Multiplexing, Journal of Laboratory Automation 2016, Vol. 21 (4) 533-547
Non Patent Literature 2
   Ryota Iino, Ultrahigh-sensitivity detection of infection/disease biomarker by a single-molecule digital ELISA, https://groups.ims.ac.jp/organization/iino_g/pdf/2013ultr aprecision.pdfSummary of Invention

### Technical Problem

However, Patent Literature 1 merely discloses a technique in which a reporter molecule serving as a labeling substance is liberated into a free liquid medium and reacted with a substrate in the liquid. This is insufficient for achieving improvement in detection sensitivity or improvement in reactivity so as to be capable of detecting a low concentration of a target substance.

Patent Literature 2 is silent about immunoassay which is performed with great sensitivity. In Patent Literature 3, the steps of a conventional ELISA in which the formation and detection of an immune complex are carried out in the same reaction field are merely implemented in a microreactor. Furthermore, a problem of the microreactor is that signal intensity is decreased with decrease in reaction capacity, though a smaller reaction field can improve a reaction rate or reactivity. Hence, in the case of carrying out immunoassay by the techniques described in Patent Literatures 2 and 3, a highly sensitive detector or camera is necessary, disadvantageously leading to a high cost. Likewise, the systems described in Non Patent Literatures 1 and 2 require a sophisticated camera or fluorescence spectroscope for fluorescence detection and therefore cannot solve the problem of a high system price.

Patent Literature 4 discloses a technique of carrying out immunoassay using a general-purpose optical disc device by establishing labeling beads to be detected. In general, the transport of granular matter in a microreactor is responsible for clogging. The technique of Patent Literature 4 causes partial accumulation of labeling beads and disadvantageously makes actual handling difficult in some cases because an antibody is immobilized on a solid-phase in a detection region and antigen-carrying labeling beads that flows to this detection region are trapped. Furthermore, the labeling beads trapped in the detection region, when coming close to each other, are disadvantageously responsible for detection errors.

In addition, in the technique described in Patent Literature 4, antigen-antibody reaction is performed between an antibody immobilized on a solid phase and an antigen carried by labeling beads while the labeling beads are travelled. Therefore, the whole detection region serves as a reaction field for the antigen-antibody reaction. Since reactivity is influenced by the frequency of contact between the antigen and the antibody, antigen-antibody reaction efficiency tends to be low in this technique in which the whole area of the detection region serves as a reaction field. Furthermore, the trapping of the labeling beads and the detection thereof (i.e., the detection of the antigen) are performed in the same reaction field. Therefore, detection sensitivity is reduced if reaction efficiency is low. Moreover, the labeling bead are difficult to be trapped by the antibody immobilized on a solid phase, depending on the traveling speed of the antibody. Therefore, unfortunately, detection sensitivity or detection rate becomes poorer.

In Patent Literatures 3 and 4, in the case of performing immunoassay, a target substance is trapped via an antibody immobilized on a detection region. Therefore, pretreatment to immobilize the antibody onto an internal detection region of a microreactor must be performed, disadvantageously leading to complicated preparation of the microreactor to be subjected to examination.

In addition, the techniques described in Non Patent Literatures 1 and 2 have a low rate of housing of introduced beads in microwells and cannot obtain sufficient sensitivity, though detection in each well can be performed. Unfortunately, this may influence the reliability of detection data.

An object of the present invention is to provide a technique that can detect a target substance in a sample with greater sensitivity and at lower cost than when using conventional technology.

### Solution to Problem

According to one aspect, the present invention provides a target substance detection method comprising:
forming a complex in a first reaction field by sandwiching a target substance between a first trapping substance immobilized on a solid phase and a second trapping substance labeled with a labeling substance;
separating a moiety that includes the labeling substance from the complex;
moving the moiety to a second reaction field; and
detecting the target substance by a signal based on the labeling substance in the second reaction field.

According to a preferred embodiment of the present invention, the solid phase is at least one selected from the group consisting of a surface, a plate-like body, a granular body, a fibrous body, a woven fabric, a nonwoven fabric, a film, and a sheet. The fibrous body means a state where fine thread-like substances exist individually or are mutually entangled to exist in floc or in a mass. The woven fabric means cloth formed by weaving a fibrous form. The nonwoven fabric means a sheet of entangled fibers without weaving. The film and the sheet each means a thin membrane directed to a form composed mainly of a polymer raw material among artificial materials. A thinner form is referred to as a film, and a thicker form is referred to as a sheet. Since the names of the film and the sheet are routinely used, the sheet includes a form produced by laminating fibrous raw materials in some cases. The woven fabric, the nonwoven fabric, and the sheet conceptually overlap .

According to another preferred embodiment of the present invention, the solid phase is a granular body and is at least one selected from the group consisting of a magnetic particle, a latex particle, and a resin particle.

According to a further alternative preferred embodiment of the present invention, the first trapping substance is at least one selected from the group consisting of an antibody, an antibody fragment, an engineered antibody, an aptamer and a nucleic acid that specifically binds to the target substance.

According to a further alternative preferred embodiment of the present invention, the second trapping substance is at least one selected from the group consisting of an antibody, an antibody fragment, an engineered antibody, an aptamer and a nucleic acid that specifically bind to the target substance.

According to a further alternative preferred embodiment of the present invention, the target substance detection method comprises removing the second trapping substance that fails to form the complex after formation of the complex.

According to a further alternative preferred embodiment of the present invention, the labeling substance is at least one selected from the group consisting of a metal colloid particle, an enzyme, a luminescent substance and a fluorescent substance.

According to a further alternative preferred embodiment of the present invention, the signal based on the labeling substance is generated in the second reaction field.

According to a further alternative preferred embodiment of the present invention, the labeling substance is an enzyme, and the signal is generated by a reaction with a substrate in the second reaction field.

According to a further alternative preferred embodiment of the present invention, the labeling substance is a metal colloid particle, and the signal is generated based on the metal colloid particle in the second reaction field.

According to a further alternative preferred embodiment of the present invention, the target substance detection method comprises carrying out an amplification reaction for amplifying the signal based on the labeling substance in the second reaction field.

According to a further alternative preferred embodiment of the present invention, the labeling substance is a metal colloid particle, and the amplification reaction includes a reaction for allowing the metal colloid particle to grow by a reduction reaction with a metal ion as a substrate.

According to a further alternative preferred embodiment of the present invention, separating the moiety that includes the labeling substance from the complex is performed by at least one treatment selected from the group consisting of heating treatment, pH adjustment treatment, oxidation-reduction treatment, enzyme treatment and competitive reaction treatment.

According to a further alternative preferred embodiment of the present invention, the signal is at least one selected from the group consisting of transmitted light, reflected light, scattered light, luminescence and fluorescence.

According to a further alternative preferred embodiment of the present invention, the second reaction field has a volume smaller than a volume the first reaction field has.

According to a further alternative preferred embodiment of the present invention, the moiety that includes the labeling substance separated from the complex contains no solid phase.

According to another aspect, the present invention provides a target substance detection device comprising at least:
a first reaction field where a complex is formed by sandwiching a target substance between a first trapping substance immobilized on a solid phase and a second trapping substance labeled with a labeling substance, and a moiety that includes the labeling substance is separated from the complex;
a second reaction field where the target substance is detected by a signal based on the labeling substance of the moiety separated from the complex in the first reaction field; and
a pathway where the moiety separated from the complex is moved from the first reaction field to the second reaction field.

According to a further alternative aspect, the present invention provides a reagent for use in a target substance detection method, the method comprising:
forming a complex in a first reaction field by sandwiching a target substance between a first trapping substance immobilized on a solid phase and a second trapping substance labeled with a labeling substance;
separating a moiety that includes the labeling substance from the complex;
moving the moiety to a second reaction field; and
detecting the target substance by a signal based on the labeling substance in the second reaction field, the reagent having an action of separating the moiety that includes the labeling substance.

According to a preferred embodiment of the present invention, the reagent comprises at least one selected from the group consisting of a pH adjuster, a denaturant, a reducing agent, an enzyme, a competing agent, and an alcohol or ester and a derivative thereof in order to perform the action of separating the moiety that includes the labeling substance.

According to another preferred embodiment of the present invention, the reagent comprises a pH adjuster in order to perform the action of separating the moiety that includes the labeling substance.

### Advantageous Effects of Invention

According to the detection method and the detection device of the present invention, a labeling substance that has formed a complex by temporarily binding to a target substance is moved to a second reaction field different from a first reaction field where the complex has been formed, and the target substance can be detected on the basis of the labeling substance. Hence, the second reaction field can be used as a suitable field dedicated to detection. Thus, the detection sensitivity of the target substance can be improved.

Furthermore, in the case of using, for example, a micro space, as the second reaction field, the target substance can be detected with greater sensitivity. Moreover, in the case of using a micro space as the second reaction field, a substance to be detected is transported via a micro flow path. In this context, according to the present invention, for example, the solid phase can be held in the first reaction field because only a portion that includes the labeling substance out of the complex bound to the solid phase has to be transferred to the second reaction field. This reduces the problem of clogging during transfer. Hence, highly sensitive detection can be appropriately carried out using a micro space such as a microreactor.

According to the reagent of the present invention, a reagent suitable for such detection can be provided.

### Brief Description of Drawings

Figure 1 is a diagram illustrating the summary of one embodiment of the detection method of the present invention.
Figure 2 is a schematic planar view showing one embodiment of the microreactor suitable for carrying out the detection method of the present invention.
Figure 3 is a diagram showing the relationship between the pH value of each reagent and cleavage characteristics in Example 1.
Figure 4 is a diagram showing the relationship between the concentration (pH value) of NaOH and cleavage characteristics in Example 2.
Figure 5 is a diagram showing the cleavage characteristics of various denaturants in Example 3.
Figure 6 is a diagram showing change in gold colloid particle size by sensitization reaction in Example 5.
Figure 7 is a diagram showing the relationship between the particle size of metal colloid particles and scattered light intensity in Example 6.
Figure 8 is a diagram showing the relationship between the concentration (which corresponds to an antigen concentration) of eluted gold colloid particles (a moiety that includes the gold colloid particles) and scattered light intensity in Example 7.

### Description of Embodiments

### <Detection method of present invention>

The detection method of the present invention comprises the steps of: (1) forming a complex in a first reaction field by sandwiching a target substance between a first trapping substance immobilized on a solid phase and a second trapping substance labeled with a labeling substance; and (2) separating (which includes the cancelation of a physical bond and the cleavage of a chemical bond) a moiety that includes the labeling substance from the complex, moving the moiety to a second reaction field, and detecting the target substance by a signal based on the labeling substance in the second reaction field.

The step (1) can be carried out, for example, by the steps of: (1-1) immobilizing a first trapping substance on a solid phase in a first reaction field; (1-2) preparing a mixed solution of a second trapping substance labeled with a labeling substance and a sample containing a target substance; and (1-3) reacting the mixed solution with the first trapping substance immobilized on the solid phase in the first reaction field to form a complex represented by solid phase-first trapping substance-target substance-second trapping substance-labeling substance. After the step (1-3), a washing step for eliminating a substance other than the complex from the first reaction field may be provided. The step (1-2) may be performed by separately preparing a sample containing a target substance and a solution containing a labeled second trapping substance, and mixing them before introduction to the first reaction field, or may be performed by introducing any one of them to the first reaction field and then introducing the other to the first reaction field. In the latter case, the step (1-3) may proceed at the same time therewith.

The step (2) can be carried out, for example, by the steps of: (2-1) separating a moiety that includes the labeling substance from the complex; (2-2) moving the separated moiety that includes the labeling substance to a second reaction field; and (2-3) detecting a signal corresponding to the quantity of the target substance trapped between the first trapping substance and the second trapping substance, by way of a signal based on the labeling substance of the moiety that includes the labeling substance in the second reaction field. Before the step (2-3), the step of mixing an agent, such as a coloring agent, necessary for generating a signal based on the labeling substance with the moiety that includes the labeling substance may be provided.

In this context, the signal to be detected is based on the labeling substance that has formed the complex by temporarily binding to the target substance. Since the quantity thereof correlates with the target substance that has formed the complex, the target substance can be detected by the present invention. The problem of clogging which arises easily when a solid phase is transferred together is reduced because only a portion that includes the labeling substance out of the complex formed by binding with the solid phase can be transferred to the second reaction field. Hence, the detection method of the present invention is suitable for detection in a micro space such as where a microreactor is used.

In the present specification, a hyphenated word, for example, "first trapping substance-target substance-second trapping substance-labeling substance", means that the components are mutually bound through a physical or chemical bond. The term "second trapping substance-labeling substance" is also described as a "second trapping substance having a labeling substance" or simply as a "second trapping substance".

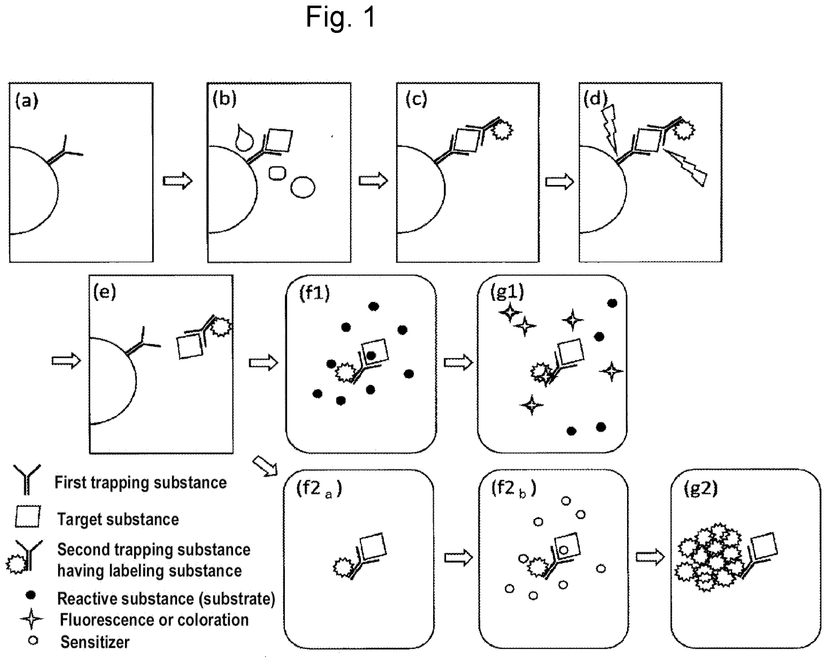

Hereinafter, one embodiment of the assay method of the present invention will be described with reference to Figure 1. Figure 1 is a schematic diagram conceptually showing one embodiment of the assay method of the present invention. For brief explanation, Figure 1 shows the case of using an antigen as a target substance and using primary antibodies as first and second trapping substances.

In an initial state, a solid phase on which a first trapping substance for trapping a target substance is immobilized is prepared in a reaction field (first reaction field) in Figure 1 (Figure 1(a)). When a sample solution containing the target substance is introduced thereto (Figure 1(b)), the target substance is specifically trapped by the first trapping substance. After a substance other than the trapped target substance is appropriately removed by a washing step, a second trapping substance labeled with a labeling substance is introduced thereto so that the second trapping substance binds to the target substance to form a complex of the target substance sandwiched between the first trapping substance and the second trapping substance on the solid phase (Figure 1(c)). An excess second trapping substance is removed to the outside of the system by a washing step. In short, the labeling substance is immobilized on the solid phase in an amount corresponding to the trapped target substance.

Then, an enzyme, an oxidation-reduction agent, a reagent consisting of an acid or a base, heat, light, vibration, or the like is applied to the formed complex (Figure 1(d)) to separate a moiety that includes the labeling substance from the solid phase. This moiety to be separated (moiety that includes the labeling substance) may be only the labeling substance or may be in a state where a molecule group is bound to the labeling substance. The molecule group is not limited to the original molecule group that remains as it is, such as a complex of first trapping substance-target substance-second trapping substance, a complex of target substance-second trapping substance, or the second trapping substance (i.e., resulting from the cleavage of an intermolecular bond), and may be a fragment obtained by the cleavage of an intramolecular bond in the original molecule group.

The separated moiety that includes the labeling substance is separated from the solid phase and liberated (Figure 1(e)), and introduced to a detection reaction field (second reaction field) indicated in the rounded-corner box in Figure 1. Here, the labeling substance (moiety that includes the labeling substance) present in the solution introduced to the detection reaction field (second reaction field) can be evaluated as the quantity of the trapped target substance. Therefore, if the presence (quantity) of the labeling substance is measured, the presence (quantity) of the target substance can be measured. In other words, in the present invention, the solid phase, the target substance or anything other than the labeling substance once bound to the target substance does not need to exist in the second reaction field where detection reaction is performed.

In the embodiment shown in Figure 1, a reactive substance that reacts with the labeling substance is also introduced to the detection reaction field (second reaction field) (Figures 1(f1) and 1(f2b)). When the labeling substance is, for example, an enzyme, a substrate is introduced as the reactive substance. As the substrate, a substance that is converted to a fluorescent substance through reaction with the enzyme, or a substance that produces color through reaction with the enzyme is selected. The resulting fluorescence or coloration is detected to measure the target substance (Figure 1(g1)).

When the labeling substance is an insoluble particle such as a metal colloid particle, change in turbidity, light scatter, or absorbance of the particles can be measured as it is to detect the target substance (Figure 1(f2ₐ)). When the labeling substance is a metal colloid particle, a reagent, such as a metal salt solution, which causes colloid sensitization reaction may be added as the reactive substance for the purpose of increasing a particle size by further forming a metal colloid particle on the surfaces of the metal colloid particle. In this case, scattered light or absorbance amplified by the growth of the colloid can be detected (Figure 1(g2)).

As mentioned above, the moiety that includes the labeling substance to be involved in detection reaction is separated from the solid phase and introduced to the detection reaction field (second reaction field). Therefore, even in the case of performing an immunoassay using a microreactor, the size of a substance that passes through a flow path can be drastically decreased, and the reactive substance can be smoothly and easily moved to the detection reaction field (second reaction field).

One embodiment of the detection method of the present invention is conceptually shown above with reference to Figure 1. However, the present invention is not limited to those described above. If the labeling substance can be detected directly by the labeling substance mentioned later, for example, if the introduction of a substrate is unnecessary or if the amplification (sensitization) of the labeling substance is unnecessary, the reactive substance that reacts with the labeling substance is unnecessary in the detection reaction field (second reaction field).

The amplification (sensitization) reaction of the labeling substance is not necessarily required to be carried out in the detection reaction field (second reaction field). The amplification (sensitization) reaction may be performed between after separation of the moiety that includes the labeling substance in the first reaction field and before introduction thereof to the second reaction field. In such a case, a reaction field where amplification reaction is performed, different from the first reaction field and the second reaction field, may be provided between the first reaction field and the second reaction field.

The first and second reaction fields will be further mentioned later.

### <Target substance>

The target substance to be analyzed in the assay method of the present invention is not particularly limited as long as the target substance can be immunologically or genetically detected. Examples thereof include pathogens such as bacteria and viruses, various proteins, peptides, nucleic acids, exosomes, and various low-molecular compounds.

### <First trapping substance>

The first trapping substance of the present invention has a function of trapping a target substance onto a solid phase and is immobilized on the solid phase for use. When the target substance is, for example, an antigen, an antibody that specifically reacts with the target substance is used. When the target substance is an antibody, an antigen with which the antibody specifically reacts is used.

In addition to the antigen and the antibody described above, a nucleic acid, an aptamer, a ligand (a substrate, an inhibitor, or an antagonist), or a receptor may be used as the first trapping substance of the present invention. When the target substance is, for example, DNA derived from a bacterium, a nucleic acid molecule complementary to the DNA can be used as the first trapping substance. When the target substance is a sequence-specific DNA-binding protein transcription factor such as a transcription factor, DNA having a particular sequence can be used as the first trapping substance.

The antibody for use as the first trapping substance may be a polyclonal antibody or may be a monoclonal antibody and is preferably a monoclonal antibody from the viewpoint of reaction specificity.

The antibody for use as the first trapping substance also includes an antibody as well as an antibody fragment and an engineered antibody having reactivity substantially equivalent to the antibody. Examples of the antibody fragment include Fab fragments, F(ab')2 fragments, Fab' fragments, and scFv fragments.

### <Second trapping substance>

The second trapping substance of the present invention binds to the target substance trapped by the first trapping substance and sandwiches the target substance between the first trapping substance immobilized on the solid phase and the second trapping substance to form a complex immobilized on the solid phase, i.e., a complex of solid phase-first trapping substance-target substance-second trapping substance (second trapping substance having the labeling substance) bound in this order. When the target substance is an antigen, an antibody that specifically reacts with the target substance is used as the second trapping substance, as in the first trapping substance. On the other hand, when the target substance is an antibody, an antigen to which the antibody specifically binds may be used. Alternatively, an antibody that binds to the antibody and is different from the first trapping substance may be used, which may be an antibody prepared in a host different from a host that produces the other antibody . Hence, when the second trapping substance is an antibody, an immune complex of solid phase-antigen (first trapping substance)-target substance-antigen (second trapping substance) or antibody (second trapping substance) or an immune complex of solid phase-antibody (first trapping substance)-target substance-antigen (second trapping substance) or antibody (second trapping substance) is formed.

In addition to the antigen and the antibody described above, a nucleic acid, an aptamer, or a specific combination of ligand-receptor such as biotin-avidin may be adopted as the second trapping substance of the present invention. In the case of, for example, DNA derived from a bacterium, a complementary nucleic acid molecule containing a target region can be used as the second trapping substance. When the target substance is a sequence-specific DNA-binding protein transcription factor such as a transcription factor, DNA having a particular sequence can be used as the second trapping substance.

The antibody for use as the second trapping substance may be a polyclonal antibody or may be a monoclonal antibody and is preferably a monoclonal antibody from the viewpoint of reaction specificity.

The antibody for use as the second trapping substance also includes an antibody as well as an antibody fragment and an engineered antibody having reactivity substantially equivalent to the antibody. Examples of the antibody fragment include Fab fragments, F(ab')2 fragments, Fab' fragments, and scFv fragments.

### <Labeling substance>

The labeling substance labels the second trapping substance and is not particularly limited as long as the labeling substance produces a certain detectable signal. The labeling substance can be, for example, an enzyme, a metal colloid particle, a fluorescent substance, a coloring substance, a luminescent substance, a fluorescent source substance, a coloring source substance, a luminescent source substance, synthetic latex such as polystyrene latex stained with a pigment, latex such as natural rubber latex, or biotin detectable with avidin bound to an enzyme or a fluorescent substance. Preferred examples of the labeling substance include enzymes, metal colloid particles, fluorescent substances, luminescent substances, coloring substances, fluorescent source substances, luminescent source substances, and coloring source substances. More preferred examples of the labeling substance include enzymes, metal colloid particles, fluorescent substances, luminescent substances, fluorescent source substances, and luminescent source substances. Further preferred examples of the labeling substance include metal colloid particles, enzymes, luminescent substances, and fluorescent substances. Still further preferred examples of the labeling substance include enzymes and metal colloid particles.

When the second trapping substance is an antibody, the antibody as the second trapping substance may have the labeling substance, or an antibody (e.g., secondary antibody) that recognizes the antibody may have a labeling substance. In the latter case, the antibody itself having the labeling substance is regarded as the labeling substance.

The binding method of the labeling substance to the second trapping substance is not limited, and a binding method, for example, binding by physical adsorption, chemical binding, binding that utilizes affinity, and a combination thereof, can be used. The labeling substance can be bound to the second trapping substance by an approach known in the art.

The signal based on the labeling substance includes a signal that is emitted from the labeling substance when the labeling substance itself is a signal source, a signal that is emitted from the labeling substance by the action of another compound when the labeling substance serves as a reactant, a signal that is emitted from another product when the labeling substance reacts as a catalyst with a separately added substrate to produce the product, and a signal that is generated through another reaction involving the labeling substance. In short, the phrase "generating a signal based on the labeling substance" or "a signal based on the labeling substance is produced" not only means that the labeling substance reacts with any of other substances to generate (produce) a signal, but includes the case where the labeling substance generates (produces) a signal by itself. In relation to this, the phrase "generating a signal based on the labeling substance" or "a signal based on the labeling substance is produced" conceptually includes not only the production of a signal through physical, chemical or biochemical reaction involving the labeling substance, but the emission of a signal by the absorption of energy by the labeling substance.

When the labeling substance is, for example, an enzyme, an enzyme substrate that can be catalyzed by the enzyme is added to the reaction system so that the substrate reacts through enzyme reaction, thereby producing a signal. The substrate can be a chromophore (coloring source substance) that produces color through enzyme reaction, a fluorophore (fluorescent source substance) that emits fluorescence through enzyme reaction, or a luminescent substance (luminescent source substance) that emits luminescence through enzyme reaction. If the labeling substance is a coloring source substance or the like, a reaction reagent (enzyme or compound) for producing a coloring substance or the like from the labeling substance as a reaction starting material (substrate) is introduced to the second reaction field to produce a signal.

In addition, for example, when the labeling substance is biotin and binds to avidin that binds to a signaling substance, the signaling substance is attached to the labeling substance, thereby generating a signal based on the labeling substance.

One example of the signal that is emitted by the absorption of energy by the labeling substance can include absorbance, absorption spectra and others of which decrease in optical physical quantity serves as a signal.

The signal generated based on the labeling substance (signal produced based on the labeling substance) is preferably a signal that can be optically detected. Specific examples of such a signal include the labeling substance and a product produced through reaction of the labeling substance, i.e., transmitted light, reflected light, scattered light, fluorescence, phosphorescence, and luminescence from a signaling substance. The labeling substance or its product can be detected by such a signal.

Examples of the fluorescent substance that may be used as the labeling substance can include fluorescein isocyanate, dyes containing a metal of europium series, and GFP protein. Examples of the luminescent substance include luciferin-luciferase luminescence systems.

The enzyme that may be used as the labeling substance (hereinafter, appropriately referred to as the a "labeling enzyme") can be various enzymes for use in an usual ELISA. Examples thereof can include horseradish peroxidase (HRP), β-galactosidase, and alkaline phosphatase.

When the second trapping substance is an antibody, a desired second trapping substance can be bound to and labeled with such an enzyme by, for example, a glutaraldehyde method, a periodic acid method or a maleimide method. Also, the enzyme may be bound to a secondary antibody of the antibody serving as the second trapping substance by the method described above, and the antibody serving as the second trapping substance can be bound and labeled with this secondary antibody. In another method, the antibody and the enzyme may be prepared as an antibody-enzyme complex expressed through binding in a genetically engineering manner. In this case, an enzyme-labeled antibody having an enzyme bound to the antibody without any inactivation of the enzyme can be obtained because there is no need of the binding of the enzyme to the antibody by the predetermined method.

Examples of the dye that may be used for colorimetrically quantifying the catalytic activity of such an enzyme can include 5-aminosalicylic acid, o-phenylenediamine, 2,2'-azinodi(3-ethylbenzothiazoline)-6'-sulfonic acid (ABTS), o-nitrophenyl-β-D-galactoside and disodium p-nitrophenylphosphate. These dyes are selected according to the type of the labeling enzyme. The dye is appropriately selected so as to be, for example, ABTS when the labeling enzyme is peroxidase (HRP), o-nitrophenyl-β-D-galactoside when the labeling enzyme is β-D-galactosidase, and disodium p-nitrophenylphosphate when the labeling enzyme is alkaline phosphatase.

For example, a dioxetane compound for alkaline phosphatase or a luminol compound for peroxidase can be used as a luminescent substrate, and 4-methylumbelliferyl phosphate for alkaline phosphatase, or resazurin or a resorufin derivative for peroxidase can be used as a fluorescent substrate, though the substrates are not limited thereto.

Various metal colloid particles can be appropriately selected and used as the metal colloid particle that may be used as the labeling substance. Examples thereof include metal colloid such as gold colloid, silver colloid, and platinum colloid, and composite metal colloid thereof. Examples of the composite metal colloid include platinum-supported gold colloid and platinum-supported palladium colloid.

In the present invention, preferred examples of the metal colloid particle include gold colloid particles, composite metal colloid particles thereof, and silver colloid particles. Gold colloid particles are more preferred.

The particle size of the metal colloid particle is usually 1 nm to 500 nm. The metal colloid particle for use in labeling is on the order of 1 nm to 500 nm. The particle size of the metal colloid particle (metal particle) for optical detection is preferably 10 nm to 10 µm. In this context, the particle size of the metal particle appropriate for detection differs depending on the sensitivity, accuracy, measurement conditions, etc. of an optical detection system selected. In the case of using, for example, general-purpose optical pickup in the detection system, the particle size is usually 100 nm to 10 µm, preferably 100 nm to 5 µm, more preferably 200 nm to 5 µm. When the particle size falls within the range of 1 µm to 3 µm, detection can be performed more easily.

In the case of using a convenient detection system that detects scattered light by a combination of laser and photodiode, the particle size of the metal colloid particle can be 100 nm or larger. When the particle size is 150 nm or larger, detection is achieved relatively easily. When the particle size is 200 nm or larger, detection is sufficiently achieved. The particle size is preferably 100 nm to 3 µm, more preferably 200 nm to 1 µm. Depending on the structure or size of the detection reaction field or measurement conditions such as a detection time, the range of 1 µm to 3 µm may be preferred in which scattered light intensity becomes stronger.

The moiety that includes the labeling substance is introduced to the reaction field (second reaction field) where detection reaction is performed. Then, a signal based on the labeling substance is detected in the second reaction field.

Although the details will be mentioned later, for example, as in the case where the labeling substance is an enzyme, a separately added substrate and the enzyme as the labeling substance may react so that a product from the substrate emits a signal. In such a case, when the second reaction field is formed of a microwell (micro volume), the probability of contact between the labeling substance and the corresponding substrate or the like is elevated. Therefore, a reaction rate is increased, and signal production efficiency is improved. Thus, a time to detection can be shortened. On the other hand, such a decreased reaction capacity also decreases the amount of the substance serving as a signal source. Therefore, the signal is attenuated.

In the case of applying a microreactor, a micro flow path is used in the transfer of a sample (moiety that includes the labeling substance) to a microwell of the second reaction field. When the labeling substance is an insoluble substance such as a metal colloid particle, a smaller particle size of the labeling substance, such as a metal colloid particle, bound to the second trapping substance is more preferred for avoiding the clogging of the flow path. In this case, the particle size of such a labeling substance is set to within a suitable range according to the width of the flow path to be formed. When the flow path diameter of the microreactor is, for example, on the order of 15 µm, the particle size of the labeling substance is preferably 200 nm or smaller and is 1 nm to 100 nm, more preferably 1 nm to 80 nm, particularly preferably 1 nm to 50 nm.

An optical signal obtained on the basis of the labeling substance is also attenuated with decrease in particle size.

In the case of an attenuated signal as mentioned above, amplification reaction is performed in a microwell, thereby improving a reaction rate while enhancing signal intensity through the amplification reaction. In association therewith, the detection thereof can be facilitated. Furthermore, the size of the labeling substance (complex bound to the labeling substance or fragment thereof) can be set to a size suitable for transfer in a micro flow path. Therefore, the transfer to the second reaction field is achieved by preventing clogging in the micro flow path. Thus, a measurement trouble can be avoided.

A method known in the art can be used as an approach of such signal amplification. When the labeling substance or a signaling substance produced on the basis thereof is, for example, a fluorescent substance, a method of enhancing fluorescence intensity or fluorescence lifetime using a photosensitizer may be used. When an enzyme is involved in signal production, for example, when the labeling substance is an enzyme, signal amplification may be performed by an enzyme cycling method using the labeling enzyme in combination with another enzyme.

A cycling reaction system known in the art can be used as an enzyme cycling reaction system. NAD cycling, CoA cycling, or ATP cycling is well known as enzyme cycling reaction known in the art. Further examples thereof can include a method of colorimetrically performing the quantification or optical detection of a protein or a nucleic acid with great sensitivity by exponentially amplifying thio NAD(P)H as a signaling substance, as described in Japanese Patent Laid-Open Nos. 4-335898 and 2014-124165.

Japanese Patent Laid-Open No. 2014-124165 discloses reaction to accumulate and amplify thio NAD(P)H in a reaction system. According to this literature, when the enzyme as the labeling substance is alkaline phosphatase (ALP), a phosphorylated substrate (androsterone derivative having a phosphoric acid group, etc.) is added such that an enzyme reaction product of the alkaline phosphatase serves as a substrate of enzyme cycling reaction. The enzyme cycling method can be carried out using 3α-hydroxysteroid dehydrogenase or the like. The enzyme as the labeling substance is not limited to alkaline phosphatase, and the enzyme cycling method can be carried out using glucosidase, galactosidase, fructosidase, mannosidase or peroxidase and using an androsterone derivative having a particular functional group or bond as a substrate of the enzyme. In this case, thio NADH or the like produced through enzyme cycling reaction serves as a signaling substance, and a signal based on the labeling substance can be detected by measuring the amount thereof as absorbance or by measuring a change in color caused by the produced thio NADH or the like.

On the other hand, when the labeling substance is a metal colloid particle, examples of the signal amplification method include sensitization reaction to allow colloid particles to grow to increase their particle sizes. As a result, the metal colloid particle to be transferred can have a micro size, and the metal colloid particle to be detected can have a large size. The metal colloid particle largely varies in the optical characteristics such as absorbance depending on the particle size. As the particle size is increased, the generated signal is enhanced and the optical detection is facilitated. Also, a change in the particle size also leads to a change in the surface plasmon characteristics. In association therewith, a wavelength of the maximum absorption is also changed. The change in the particle size enables the optical measurement of the wavelength to be performed in response to the maximum absorption and enables the optical detection to be performed more easily. As a result, smooth transport of a reactive substance (labeling substance) in a microreactor and improvement in detection sensitivity can both be achieved. The sensitization reaction can be performed by a method described in, for example, Japanese Patent Laid-Open No. 2009-192270.

In this context, in the present invention, in the case of using, for example, metal colloid as the labeling substance, metal ions in a metal salt solution containing gold ions or silver ions separately added to the reaction system may be reduced and thereby accumulated on the metal colloid surface of the labeling substance through the use of the catalytic activity of the metal colloid or by combined use with a reducing agent so that the particle size of the metal colloid is increased, thereby enhancing a detectable signal.

A core metal species having high stability against oxidation-reduction is preferred for the metal colloid particle that responds to sensitization action. The core metal colloid particle may be formed from a single metal or may be a composite metal. Examples of such metal colloid particles include metal colloid such as gold colloid and platinum colloid, and composite metal colloid thereof as well as synthetic latex such as polystyrene latex stained with a pigment such as a red or blue pigment, and latex such as natural rubber latex, as the labeling substance. Among them, metal colloid such as gold colloid is particularly preferred. Examples thereof include gold colloid particles, platinum colloid particles, and palladium colloid particles. Examples of the composite metal colloid particles include platinum-gold colloid particles composed of platinum colloid particles carried on gold colloid particle.

Such a metal colloid particle can be produced by a method known in the art. A gold colloid particle can be prepared, for example, by reducing an aqueous tetrachloroauric acid (HAuCl₄) solution with a reducing agent such as citric acid under boiling. Also, a platinum-gold colloid particle can be produced by further reducing chloroplatinic acid (H₂[PtCl₆]) with a reducing agent such as citric acid or ascorbic acid in the presence of the prepared gold colloid particle.

The sensitization reaction of such a metal colloid particle can be performed by precipitating a metal through reduction reaction from a solution containing a metal ion, and accumulating the metal on the metal colloid particle surface, thereby increasing the particle size of the metal colloid. Examples of the metal salts that is used as a supply source of metal ions include salts of metals that belong to the 4, 5, and 6 groups in the periodic table. It is preferred to use a salt of gold, platinum, silver, palladium, nickel, cobalt, copper, or the like. The metal to be precipitated may be the same type of metal or a different type of metal with or from the metal colloid particle and is not limited as long as reduction reaction can be carried out under simple conditions.

For example, palladium-platinum colloid particles can be allowed to grow by using a solution containing any metal ions selected from nickel ion, cobalt ion, and copper ion as a sensitizer solution.

For example, gold colloid particles can be allowed to grow by using a sensitizer solution containing chloroauric acid as a supply source of gold ions, and accumulating gold colloid on the surfaces of the gold colloid particles as the labeling substance. Also, the gold colloid particles can be allowed to grow as composite metal colloid particles by using a sensitizer solution containing silver nitrate, and accumulating silver colloid on the gold colloid particle surfaces. Platinum-gold colloid particles can be allowed to grow using a sensitizer solution containing silver nitrate or a copper salt such as copper sulfate through the use of the catalytic activity of platinum.

The sensitizer solution contains any of various metal salts serving as a supply source of the metal ions as an essential component and can optionally further contain a complexing agent, a pH adjuster, a buffer, a stabilizer, and the like. A conventional non-electrolytic plating solution known in the art may be used as such a sensitizer solution.

Examples of the complexing agent include ammonia, citrate, tartrate, and lactate. Examples of the pH adjuster include acetate, propionate, and ammonium salt. Examples of the stabilizer include various surfactants.

A solution containing a reducing agent may be used for accelerating the reduction reaction of the metal ions. Examples of the reducing agent include, but are not limited to, citric acid, ascorbic acid, sodium hypophosphite, hydrazine monohydrate, hydrazine sulfate, sodium borohydride, dimethylamine borane, formaldehyde, Rochelle salt, glucose, and ethylene glycol. The reducing agent to be used is appropriately selected so as to be suitable for the metal ion to be reduced.

The sensitization reaction may be performed with a sensitizer solution containing both the metal ion component and the reducing agent, or a metal salt solution containing the metal ion component and a reducing agent solution containing the reducing agent may be mixed immediately before sensitization reaction. Particularly, a method of separately adding the metal salt solution and the reducing agent solution to the reaction system is preferred. Depending on the catalytic activity of the metal colloid particle, the sensitization reaction can be carried out without the addition of the reducing agent solution.

### <Method for cutting out moiety that includes labeling molecule>

In the detection method of the present invention, after a complex of first trapping substance-target substance-second trapping substance (second trapping substance having the labeling substance) is formed on the solid phase in the first reaction field, a moiety that includes the labeling substance is cut out (separated) from this complex and liberated into a solution from the solid phase. As described above, this separated moiety only has to include the labeling substance but does not need to be in the state of the whole second trapping substance including the labeling substance. In short, the separation may be made by the cleavage of the bond between the labeling substance and the second trapping substance, or the bond between the solid phase and the first trapping substance may be cleaved. Any bond within the complex may be cleaved as long as the labeling substance or the moiety that includes the labeling substance is separated. Preferably, the moiety that includes the labeling substance is a moiety that includes the labeling substance separated from the formed complex, more preferably a moiety that includes the labeling substance without including the first trapping substance, further preferably only the labeling substance without including the first trapping substance and the second trapping substance.

As described above, the binding method of the labeling substance to the second trapping substance is not limited, and a binding method, for example, binding by physical adsorption, chemical binding, binding that utilizes affinity, and a combination thereof, can be used. The labeling substance can be bound to the second trapping substance by an approach known in the art.

Examples of the approach of cleaving (separating) the moiety that includes the labeling substance can include a cleavage method of applying physical energy such as heat, light, or vibration from the outside, and a method of adding a cleavage reagent comprising an oxidizing agent, a reducing agent, or the like. The cleavage reagent may cleave the bond between the solid phase and the first trapping substance or may cleave other bonds within the complex. Preferably, the cleavage reagent can separate a moiety that includes the label from the complex formed on the solid phase, and particularly preferably cleaves the bond between the target substance and the second trapping substance or between the second trapping substance and the labeling substance.

Examples of the cleavage reagents that cleaves the bond include pH adjusters, denaturants, reducing agents, enzymes, competing agents, and alcohols or ester and derivatives thereof, and preferably include pH adjusters, denaturants, reducing agents, enzymes, and competing agents, more preferably pH adjusters, denaturants, reducing agents, and enzymes.

The pH adjuster is a compound or a composition that contains an acid, a base, or a salt thereof and changes pH. The acid may be any of inorganic acids and organic acids, and the base may be any of inorganic bases and organic bases. Examples of such an acid and a base include, but are not limited to, hydrochloric acid, boric acid, carbonic acid, nitric acid, acetic acid, boronic acid, formic acid, citric acid, phosphoric acid, oxalic acid, lactic acid, malic acid, salicylic acid, glycine hydrochloride, glycine-sodium hydroxide, sodium hydroxide, amines such as triethylamine, dimethylamine, and methylamine, ammonia, sodium bicarbonate, and salts thereof. Among them, strong acids and strong bases are preferred. Specific examples thereof include hydrochloric acid, nitric acid, formic acid, phosphoric acid, oxalic acid, glycine hydrochloride, glycine-sodium hydroxide, sodium hydroxide, and amines such as triethylamine, dimethylamine, and methylamine.

The denaturant is a reagent that changes the higher-order structure of a protein or a nucleic acid. Examples thereof include, but are not limited to, protein denaturants such as urea and guanidine salt, and surfactants such as sodium dodecyl sulfate.

Examples of the reducing agent include 2-mercaptoethanol and dithiothreitol which cleave a disulfide bond.

The enzyme hydrolyzes part of a component of the complex. Examples thereof include pepsin, papain, ficin, nuclease, and enzymes that specifically acts on a particular bond of the component.

Examples of the competing agent including agonists, antagonists, and competitive antibodies which compete with the target substance for binding to the first or second trapping substance.

The type or concentration of the cleavage reagent is appropriately selected according to the structure or binding force of the complex concerned, and adjusted without inactivating the functions of the labeling substance.

In this context, a linker may be introduced to a predetermined portion of the complex in advance such that a desired portion of the complex is specifically cleaved. This linker, for example, when the solid phase and the first trapping substance or the labeling substance and the second trapping substance are bound to each other, may be used for the purpose of linking these two substances concerned. As long as the linker has a predetermined functional group or a reactive group that can form chemical bonds at both ends, the substances concerned such as the labeling substance and the second trapping substance can be linked via the functional groups at both ends of the linker. The linker can be used for the purpose of, but not limited to, linking the solid phase and the first trapping substance or the labeling substance and the second trapping substance. Examples of the functional groups or the reactive groups at both ends of the linker include groups that form covalent bonds such as an amide bond, a disulfide bond, or an ester bond. In this case, the complex can be cleaved by the hydrolysis or reducing agent treatment of the terminal binding sites of the linker.

The mechanism of binding between the linker and the substance concerned is not limited to the covalent bond, and may be binding, for example, physical adsorption, ionic binding, binding that utilizes intermolecular affinity such as biotin-avidin, and a combination thereof.

The molecular structure constituting the linker is not limited, and a protein, a peptide, a sugar chain, a nucleic acid, or a synthetic polymer such as polyethylene glycol can be used. Alternatively, for example, biotin-avidin which forms affinity binding may be used. In this case, the linker can be treated with an enzyme such as endopeptidase, glycolytic enzyme, or endonuclease, an acid, a surfactant, or the like to cleave the linker midway.

Such a linker moiety can be cleaved by physical energy from the outside or with a cleavage reagent such as an enzyme.

Specifically, for example, if a disulfide bond is cleaved, this bond can be cleaved by the action of a reducing agent having a thiol group in the molecule, such as 2-mercaptoethanol or dithiothreitol. As for a biotin-avidin bond, the bond can be cleaved by changing the pH of the reaction system by the addition of a pH adjuster, or by using an elution buffer. When the linker molecule is constituted by a sugar chain, a particular bond can be cleaved by allowing a specific enzyme to act on the bond between constituent monosaccharides.

In the case of using a linker having a histidine tag, for example, a linker molecule having a terminal histidine tag is bound to the first trapping substance, and nickel derivatized with a chelating agent is immobilized on the surface of the solid phase. The first trapping substance can thereby be immobilized on the solid phase via the histidine tag. When histidine or imidazole which is a competing agent against the histidine tag is added thereto as a cleavage reagent, the histidine tag can be dissociated from the nickel. Therefore, the complex containing the labeling substance can be separated from the solid phase.

The cleavage reagent mentioned above may be removed or inactivated after addition. For example, a treatment is mentioned in which when the reaction field is shifted from neutrality to acidity or alkalinity by the addition of a pH adjuster, neutrality is recovered by the further addition of a pH adjuster.

### <Solid phase>

The solid phase is an insoluble solid to which the first trapping substance binds. The solid phase is a scaffold to which the complex binds in the first reaction field, and is processed into a form, a shape, or a size that enables the solid phase to be held in the first reaction field.

Examples of the form or the shape of the solid phase include, but are not particularly limited to, surfaces (surfaces having a predetermined area, such as reaction container surfaces, i.e., the inner surfaces of reaction containers), plate-like bodies, granular bodies, fibrous bodies, woven fabrics, nonwoven fabrics, films, and sheets which are housed in reaction containers. The plate-like body, the granular body and the fibrous body may be in any form such as a hollow, solid, or porous form. The shape of the granular body can be selected from various shapes such as a globe, an annular body, a flat body, a columnar body, and an amorphous body. The granular body can be, for example, at least one selected from a magnetic particle, a latex particle, and a resin particle.

A binding manner, for example, physical adsorption, covalent binding, ionic binding, binding that utilizes affinity, or a combination thereof, can be used in the binding between the solid phase and the first trapping substance. Preferably, the first trapping substance is immobilized on the solid phase by covalent binding or ionic binding which is stronger binding than binding force ascribable to affinity force or adsorptive force.

A solid phase having a large specific surface area is preferably used as such a solid phase. For example, a plate-like body, a granular body, a fibrous body, a woven fabric, or a nonwoven fabric is preferably used, and a granular body, a fibrous body, a woven fabric, or a nonwoven fabric is more preferably used. Further preferably, a granular body is used. Since a large specific surface area is preferred, a smaller particle size is more preferred for the plate-like body and the granular body.

### <Reaction field>

The target substance detection method according to the present invention involves introducing a sample to a first reaction field having a solid phase on which a first trapping substance is immobilized in advance, reacting a target substance in the sample with the first trapping substance on the solid phase, and after a washing operation, introducing a second trapping substance thereto to form a complex of the target substance sandwiched therebetween. Subsequently, a washing operation is performed to discharge a redundant second trapping substance to the outside of the reaction system. Then, as mentioned above, a bond within the complex is cleaved to separate a moiety that includes the labeling substance. These steps up to the separation of the moiety that includes the labeling substance are performed in the first reaction field.

The first reaction field is not particularly limited as long as the field enables a complex of solid phase-first trapping substance-target substance-second trapping substance (second trapping substance having the labeling substance) to be formed and a moiety that includes the labeling substance to be separated from this complex.

It is preferred that the moiety that includes the labeling substance separated from the complex should include no solid phase, from the viewpoint of deliverability, etc. Thus, the moiety that includes the labeling substance is preferably liberated from the complex by the separation between the solid phase and the first trapping substance, of the first trapping substance midway, between the first trapping substance and the target substance, of the target substance midway, between the target substance and the second trapping substance, of the second trapping substance midway, or between the second trapping substance and the labeling substance.

For example, a test tube, a microtube, a microplate, an internal chamber of a microreactor, or a bio disc can be used as the first reaction field. The operation in the first reaction field may be manually performed or may be automatically performed. In the case of automating the operation in the first reaction field, the first reaction field may be constituted by, for example, a device implemented in the form of a microreactor and may be constituted in the form of a disc.

The second reaction field is located at a position different from the first reaction field, and is a reaction field to which the moiety that includes the labeling substance separated in the first reaction field is introduced. The second reaction field is a field where, as described above, the target substance is detected by a signal based on the labeling substance. In the first reaction field, components other than the complex are discharged to the outside of the system by washing, and then, the step of cleaving the moiety that includes the labeling substance is performed. Hence, in the second reaction field, impurities that inhibit measurement are reduced, and the moiety that includes the labeling substance is introduced in a highly pure state. A signal based on the labeling substance is produced in the second reaction field.

The second reaction field is preferably formed with a micro volume (microwell). The second reaction field is particularly preferably formed with a volume equal to or smaller than the reaction volume of the first reaction field. The second reaction field may take a form of various reaction containers. Preferably, a plurality of microwells are established, and a solution containing the moiety that includes the labeling substance and is separated in the first reaction field is dispensed to the plurality of wells. Among the plurality of microwells, a signal is detected from a microwell where the moiety that includes the labeling substance is housed, while no signal is detected from a microwell where the labeling substance is not housed. Thus, the "presence" or "absence" of the signal from the microwells can be counted to clearly determine the presence of the target substance in the sample. The concentration of the target substance can be approximately calculated from the obtained detection results by adopting a limiting dilution technique and making a correction on the precondition that the dispensing of the labeling substance to the microwells follows Poisson distribution.

In the case of performing chemical reaction (including biochemical reaction) involving the labeling substance in the second reaction field, the second reaction field formed with microwells can enhance reaction efficiency and can accordingly improve a reaction rate. Hereinafter, the case of carrying out chemical reaction involving the labeling substance in the second reaction field will be described in more detail.

The volume of the microwell is designed in consideration of a reaction rate and the intensity of a signal to be generated. The volume of each microwell can be configured in the range of 1 × 10⁻⁸ to 100 µL.

The number of microwells is designed as one or more and preferably designed in the range of 10² to 10⁷. The volume and the number of microwells are appropriately designed such that the total volume of all the microwells as the second reaction field is larger than the total amount of the solution containing the moiety that includes the labeling substance separated in the first reaction field. A plurality of microwells may be established in an array pattern.

The microwells can be formed at a number that can accommodate the total amount of the sample solution of the first reaction field. The volume of the reaction field can thereby be smaller than that of the first reaction field according to the number. For example, the second reaction field can have 1/10² to 1/10⁷ of the volume of the first reaction field according to the number of microwells.

The array can assume various shapes. Examples thereof include round, triangular, quadrangular (e.g., square), regular hexagonal, and regular octagonal shapes. The array may be in the form of a microplate or in a flat circular shape (disc shape).

A substance that specifically binds to the moiety that includes the labeling substance may be immobilized on the microwells. For example, the moiety that includes the labeling substance to be separated is biotinylated in advance, and the solution is introduced to the microwells on which avidin is immobilized in advance. As a result, the moiety that includes the target substance can be efficiently held within the microwells.

As mentioned above, a plurality of microwells are established. As a result, the solution containing the moiety that includes the labeling substance separated in the first reaction field is dispensed to these microwells, and reaction can be carried out with high efficiency in the micro space. Hence, the presence of a target substance can be more accurately detected even if the target substance in the sample has a low concentration.

The second reaction field may be configured in a form where a plurality of microwells are housed in a reaction device, in order to perform reaction and signal detection in the microwells.

### <Reaction device>

The reaction device is a reaction device called microreactor. The reaction device is configured to have, in the inside thereof, microwells and a supply flow path which communicates with the microwells while leading to the outside via a supply port. Also, the reaction device can be appropriately provided with a discharge port for discharging a waste liquid to the outside of the device or a reservoir where a waste liquid is pooled, and a discharge flow path which transfers a waste liquid from the microwells to the discharge port or the reservoir. The microwells and the supply flow path are formed inside the reaction device. A solution and a reagent to be introduced from the first reaction field, an oil for sealing the microwells, and the like are supplied from the supply port into the reaction device.

In the reaction device, the individual microwells are arranged along a flow path. In such a case, each of the microwells is disposed as a recess so as to communicate with the flow path. When the reaction device is formed in a disc shape mentioned later, the microwells are disposed as recesses lateral to the flow path and on the outer circumferential side of the disc.

The flow path to be established in the reaction device is formed with a large dimension compared with the microwells. The flow path can be appropriately designed according to the volume of the microwells. For example, the flow path width is designed in the range of 1 to 1000 micrometers, and the flow path depth is appropriately designed in the range of 1 to 1000 micrometers. When the flow path width is formed as a sufficient width compared with the size of the labeling substance contained in the solution to be introduced from the first reaction field, the moiety that includes the labeling substance can be smoothly transferred.

The transfer of the sample solution within the reaction device can be performed by pressure sending using a pump or a pipette. The transfer of a fluid may be performed through the use of rotational force and centrifugal force that are generated by forming the microreactor in a disc shape and rotating the disc.

In the case of forming the reaction device in a disc shape, the shape of the flow path is not particularly limited as long as the sample solution can be conveyed by rotational force or centrifugal force. The flow path may be radially formed from near the central part of the disc toward the outside, or a whorl-like flow path may be formed. Alternatively, the flow path may be formed into concertinas or branched. In the case of forming a whorl-like flow path, a plurality of flow paths may be concentrically formed, or only one flow path may be formed. Alternatively, the flow path may partially have a whorl-like shape, or a plurality of whorl-like flow paths may be formed. The supply port is established on the center side of the disc in order to convey the solution, etc. introduced into the reaction device by centrifugal force or rotational force.

The shape and number of microwells and the flow path width are appropriately designed such that the microwells disposed in the reaction device can be filled with the solution introduced from the first reaction field, a separately added reagent, or the like by performing the operation at the number of rotations of 500 to 7,000 rpm for 1 second to 10 minutes in order to obtain the centrifugal force or the rotational force for feeding the solution, etc. introduced from the supply port.

In the case of designing the second reaction field as a plurality of microwells, it is desirable that: the amount of the solution containing the moiety that includes the labeling substance introduced from the first reaction field should be set to a capacity smaller than the total volume of the plurality of microwells that form the second reaction field; and the amount of the solution should fill 10% or more, preferably 30% or more, more preferably 50% or more, further preferably 70% or more, particularly, 90% or more, of the total number of microwells as the second reaction field formed to communicate with the flow path.

When the amount of the solution supplied from the first reaction field is excessive compared with the total volume of the microwells constituting the second reaction field, a reservoir where a solution or a waste liquid is pooled may be established at the end of the flow path. Alternatively, the flow path may be configured such that an excessive sample solution flows out of its end.

The reaction device can be prepared by a method known in the art using a material known in the art such as various plastics (e.g., acrylic resin, polycarbonate resin, and silicon resin) or an inorganic material (e.g., glass and silicon). It is preferred that the material should be transparent, for macroscopically or optically observing the situation of the flow path and the wells.

The reaction device can also be prepared by use of an approach known in the art. The reaction device can be produced, for example, by laminating a sheet or a plate having patterns of a flow path and microwells to a flat sheet or plate having a supply part for a sample solution. The reaction device may be formed by machining or etching using photolithography. A reflective layer may be formed in the microwells or on the undersurface thereof in order to increase the light quantity of an incident signal from the microwells to a detector.

The signal based on the labeling substance is detected by a predetermined detection means in the second reaction field. A detection means that can detect the signal in a noncontact manner is preferably used. For example, an optical sensor such as a CCD camera, a CMOS camera, optical pickup, or photodiode can be used for detecting transmitted light, reflected light, scattered light, fluorescence, phosphorescence, or luminescence from a signaling substance.

The scattered light can be measured with a detection system having laser and photodiode. Although optical pickup also has laser and photodiode, the optical pickup measures reflected light (return light) in the direction of incident light from a subject, i.e., absorbance. On the other hand, the scattered light detection system detects light scattered in a direction different from the direction of incident light, and differs in this point from the optical pickup.

Scattered light intensity is increased in proportion to, for example, the concentration of metal (colloid) particles in a solution. Therefore, the concentration of the metal (colloid) particles in the solution can be measured by measuring the scattered light intensity. The scattered light intensity, when the particle size of the metal (colloid) particles falls within a predetermined range, correlates with the particle size. Also, scattered light intensity in a particular direction varies depending on the particle size. Accordingly, in the case of establishing a scattered light detection system as a detection means, the signal based on the labeling substance can also be detected.

In the production of the signal based on the labeling substance in the second reaction field, a reagent for producing the signal based on the labeling substance may be introduced, together with the solution containing the moiety that includes the labeling substance, to the microwells, or such a reagent may be introduced to the microwells separately from the solution containing the moiety that includes the labeling substance. In the case of further carrying out the amplification reaction of the signal, a mixture of reagents necessary for signal amplification may be introduced to the microwells, in addition to those described above, or these reagents may be separately introduced to the microwells. For example, when a sensitizer solution is composed of a plurality of solutions, these solutions may be introduced in a mixed state to the microwells or may be separately introduced thereto.

For such mixing, after the mixing of the solution containing the moiety that includes the labeling substance and other reagents (substrate that reacts with the labeling substance, enzyme, and other reactive substances) is carried out using another chamber, the mixed solution may be introduced to the microwells. Alternatively, the reagents may be mixed into a flow path before introduction of the solution containing the moiety that includes the labeling substance from the first reaction field to the microwells serving as the second reaction field.

The reaction rate between the labeling substance and a substrate, etc. can be adjusted by appropriately adjusting reagent composition or a substrate concentration. Hence, the reaction between the labeling substance and a reagent or a component in the reagent can be prevented from occurring during liquid transfer. After they are introduced to the microwells, the reaction rate is drastically increased. Therefore, the reaction of interest can be appropriately carried out.

Such reagents may be configured such that reaction is started by applying thereto energy from the outside, such as heat.

Some reagent components may be arranged in a dry state in the microwells or the flow path in advance. For example, a metal salt component to be contained in a sensitizer solution and a reducing agent are each arranged in a dry state in the flow path between the first reaction field and the second reaction field. Each reagent is dissolved by the solution introduced into the flow path to prepare a sensitizer solution and a reducing agent solution. While reaction with the labeling substance contained in the solution is allowed to proceed, the solution can be introduced to the microwells. In another embodiment, the metal salt component and the reducing agent may be arranged in a dry state in the microwells in advance, and dissolved by the introduction of the solution containing the labeling substance to start sensitization reaction.

When the first reaction field described above is constituted in the form of a disc, a first disc constituting the first reaction field and a second disc constituting the second reaction field may be overlaid to integrally form the reaction device. In such a case, the second disc is designed so as to face the detector side. A communication port is further formed such that the sample solution can be transferred from the first disc to the second disc.

### <Target substance detection method 1 (antibody examination)>

In an exemplary target substance detection method 1 according to the present invention, an antigen against a target substance is immobilized on a polystyrene bead surface. Subsequently, blocking is performed, and then, a chamber having a predetermined capacity is filled with the polystyrene beads. A sample, such as serum, containing the target substance (antibody) is introduced thereto and reacted by contact with the beads for a given time, followed by washing with a buffer. Subsequently, a secondary antibody (labeled antibody) solution is introduced thereto and reacted for a given time. An immune complex of antigen-antibody-secondary antibody in a sandwiched manner is thereby formed on the beads. After washing with a buffer, a cleavage reagent (pH adjuster) is introduced so as to attain pH 2 to 4 so that a moiety that includes the labeling substance is liberated from the immune complex formed on the solid phase.

The chamber is provided with an elution port having a smaller size than a filter or bead diameter. After cleavage treatment, a solution containing the labeling substance is taken out of the elution port. Then, a reagent for producing a signal based on the labeling substance in the solution is mixed into the solution. When the labeling substance is a signaling substance itself, such as a coloring substance, a fluorescent substance, a luminescent substance, or metal colloid particles, such a reagent is unnecessary. When the labeling substance is an enzyme, a substrate that develops fluorescence, luminescence or color through enzyme reaction is mixed thereinto. When the labeling substance is biotin or the like, a signaling substance bound to avidin is mixed therewith. When pH influences the reaction, for example, when an enzyme participates in signal production, the pH is readjusted to near 7 with a neutralizing agent after introduction of the cleavage reagent. Then, signal production reaction is carried out.

The elution port may be provided with an openable and closable cover. The chamber may be configured such that the solution flows out of the elution port by pressing the solution in the chamber.

Then, a reagent for signal amplification is mixed therewith, if necessary, and then, the solution is introduced to a reaction device provided with a plurality of microwells, and dispensed to the microwells. After a lapse of a predetermined time or immediately thereafter, a signal from the microwells is detected using an optical unit.

Immunoassay of detecting the target substance can be carried out by such a method.

### <Target substance detection method 2 (antigen examination)>

In exemplary target substance detection method 2 according to the present invention, immunoassay called sandwich scheme can be carried out. First, an antibody against a target substance is diluted into an appropriate concentration using phosphate-buffered saline (hereinafter, referred to as PBS) or a buffer solution and immobilized on polystyrene bead surfaces. Subsequently, blocking is performed, and then, a chamber having a predetermined capacity is filled with the polystyrene beads. A sample, such as serum, containing the target substance (antigen) is introduced thereto and reacted by contact with the beads for a given time, followed by washing with a buffer. Subsequently, a labeled primary antibody is introduced to the chamber and bound to the antigen. An immune complex of antibody-antigen-antibody in a sandwiched manner is thereby formed on the beads. Then, immunoassay of detecting the target substance can be carried out by the same procedures as in the detection method 1 mentioned above.

Hereinafter, preferred embodiments of the present invention will be further described with reference to the attached drawings.

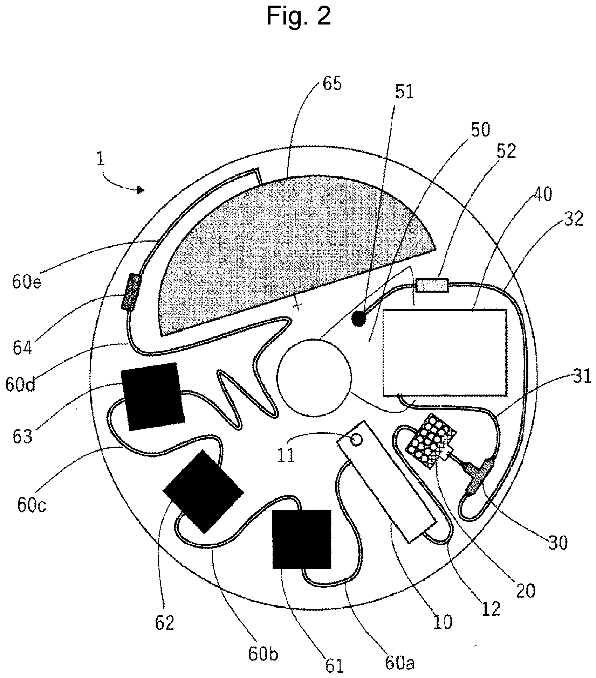

The detection method of the present invention can be carried out using, for example, a device shown in Figure 2.

Figure 2 shows a microreactor formed in plastic disc 1 in a flat circular shape, such as a compact disc (CD),a DVD, or a Blu-ray disc (BD). Reference numeral 10 denotes a thin, long sample reservoir for receiving and temporarily housing a predetermined amount of a test sample, and has sample addition port 11 which opens upward from the disc 1 at an end part on the inward side of the disc 1 (hereinafter, simply referred to as the "inward side"). A retaining member impregnated with a second trapping substance labeled with a labeling substance is housed inside the sample reservoir 10. Reference numeral 20 denotes a trapping region filled with a solid phase such as polystyrene beads on which a first trapping substance is immobilized, and forms a first reaction field. An end part of the sample reservoir 10 on the outward side of the disc 1 (hereinafter, simply referred to as the "outward side") communicates with an end part on the inward side of the trapping region 20 via flow path 12. The outward end part of the trapping region 20 communicates with the inlet of T-shaped branch tube 30 which is branched in opposite directions along the circumference of the disc 1. An outlet of the branch tube 30 branched in a counterclockwise direction of the disc 1 communicates with waste liquid tank 40 via flow path 31, while an outlet thereof branched in a clockwise direction of the disc 1 communicates with outlet 51 via flow path 32. The T-shaped branch tube 30 is provided in the inside thereof with a valve which switches the inflow direction of a liquid. Such a valve switches an internal plug between a blocked state and a released state, and various valves that can be operated by electricity, magnetic force, electromagnetic force, a machine mechanism, inertial force, centrifugal force, or heat can be used. Examples of the plug to be established in the inside can include metal pieces, beads, air, magnets, thermal melting resin, and grease. Preferably, a desirable plug has a mechanism of moving within the branch tube by the centrifugal force, allowing the position of the blocking being switched (e.g., beads and air).

The outlet 51 communicates with a disc for sample analysis having a second reaction field 50 established in the lower layer of the disc 1. A sample solution sent out of the first reaction field is introduced to the disc for sample analysis via a flow path (not shown) from the outlet 51 and transported to the second reaction field. A reading device for an optical disc as described in, for example, Japanese Patent Laid-Open No. 2012-255772 or Japanese Translation of PCT International Application Publication No. 2002-530786 is arranged beneath the disc 1 so as to be able to detect a signal from the second reaction field.

One end of flow path 60a is connected to near the end part on the inward side of the sample reservoir 10, and first liquid tank 61, second liquid tank 62, third liquid tank 63, fourth liquid tank 64, and fifth liquid tank 65 communicate therewith in this order from the sample reservoir 10 via flow paths 60a, 60b, 60c, 60d, and 60e. A washing solution for washing off a substance other than a complex comprising a complex (i.e., -first trapping substance-target substance-second trapping substance (including the labeling substance)) formed in the trapping region 20 and immobilized on the solid phase is housed in the first to third liquid tanks 61 to 63. In the present embodiment, three liquid tanks for pooling the washing solution are established. However, such a liquid tank is not limited thereto, and one or more liquid tanks can be established.

A cleavage reagent for cutting out only a moiety that includes the labeling substance from the complex (i.e., first trapping substance-target substance-second trapping substance (including the labeling substance) immobilized on the solid phase) formed in the trapping region 20, and discharging this moiety from the outward end part of the trapping region 20 to the branch tube 30 is pooled in the fourth liquid tank 64.

An operating solution for pressing liquids housed in the first to fourth liquid tanks 61 to 64 so as to flow out toward the direction of the trapping region 20 is housed in the fifth liquid tank 65. A solution immiscible with the sample solution is usually selected as the operating solution.

The flow paths 60a, 60b, 60c, 60d, and 60e are filled with air, which is in turn sent to the downstream side in association with the pressing of the liquid from the fifth liquid tank 65. A sensor (not shown) is established between the trapping region 20 and the flow path 12 so as to be able to detect whether an object passing through the flow path is air or a liquid. The sensor can detect the passage of the liquid and can recognize which liquid tank the liquid flows from.

At the time of detection, a sample solution is injected from the sample addition port 11 to fill the sample reservoir 10. In the sample reservoir 10, the sample solution is contacted with the second trapping substance labeled with the labeling substance. Then, the disc 1 is rotated in a counterclockwise fashion. As a result, the operating solution housed in the fifth liquid tank 65 flows out to the flow path 60e by centrifugal force ascribable to the rotation of the disc 1, and presses the liquids housed in the first to fourth liquid tanks 61 to 64 and air in each of the flow paths 60a, 60b, 60c, 60d, and 60e so that the sample solution housed in the sample reservoir 10 flows into the trapping region 20 from its end part on the inward side via the flow path 12. In this respect, provided that the target substance is contained in the sample solution, a complex comprising solid phase-first trapping substance-target substance-second trapping substance (including the labeling substance) is formed in the trapping region 20.

Upon further counterclockwise rotation of the disc 1, the washing solutions housed in the first to third liquid tanks 61 to 63 flow into the trapping region 20 through the sample reservoir 10 and discharge a substance other than the complex formed in the trapping region 20 to the waste liquid tank 40 via the branch tube 30 and the flow path 31 from the trapping region 20. Then, the cleavage reagent housed in the fourth liquid tank 64 flows into the trapping region 20 through the sample reservoir 10 and cuts out only a moiety that includes the labeling substance from the complex formed in the trapping region 20. When the inflow of the cleavage reagent to the trapping region 20 is detected by the sensor, the rotation of the disc 1 is suspended and in turn, the disc 1 is rotated in a clockwise fashion. The solution containing the cut-out moiety that includes the labeling substance in the cleavage reagent is thereby transferred to the second reaction field 50 via the branch tube 30 and the flow path 32 from the trapping region 20. If a coloring agent or a sensitizer is placed in space 52 in the middle of the flow path 32, the moiety that includes the labeling substance in a state mixed with the coloring agent or the sensitizer is transferred to the disc for sample analysis in the second reaction field 50, and the labeling substance generates a detectable signal in the second reaction field 50. In this state, therefore, the signal can be detected using an appropriate reading device.

### Examples

Hereinafter, the present invention will be described in more detail with reference to Examples. However, the present invention is not limited to the description of Examples.

### (Reference Example 1) Preparation of platinum-gold colloid suspension

All glass instruments to be used were washed with aqua regia. 390 mL of ultrapure water was placed in a flask and boiled. To this boiled water, 30 mL of an aqueous chloroauric acid solution (1 g of gold per L of aqueous solution; manufactured by Katayama Chemical Industries Co., Ltd.) was added, and then, 60 mL of a 1 wt% aqueous sodium citrate solution was added. After the addition of the aqueous sodium citrate solution, several tens of mL of an aqueous chloroplatinic acid solution (1 g of platinum per L of aqueous solution; manufactured by Wako Pure Chemical Industries, Ltd.) were added thereto. 5 minutes later, 60 mL of a 1 wt% aqueous sodium citrate solution was added thereto, and the mixture was refluxed for 4 hours to obtain a platinum-gold colloid suspension. Before use in each Example, the particle size of the colloid was adjusted to 100 nm or larger by appropriate addition of a reagent or a reducing agent so as to be able to accurately detect the separation of a moiety that included the labeling substance.

### (Reference Example 2) Preparation of monoclonal antibody

Using a commercially available SARS-CoV-2 nucleocapsid protein (hereinafter, simply referred to as "SARS-CoV-2NP") (CUSABIO Technology LLC) as an antigen for immunization, an anti-SARS-CoV-2NP monoclonal antibody (hereinafter, simply referred to as an "anti-NP antibody") was obtained in accordance with a routine method.

Specifically, a mouse (BALB/c, 5 weeks old, Japan SLC, Inc.) was immunized three times with 100 µg of SARS-CoV-2NP, and its spleen cells were used in cell fusion. Sp2/0-Ag14 cells (Shulman et al., 1978), mouse myeloma cells, were used in the cell fusion. A culture solution of Dulbecco's Modified Eagle Medium (Gibco) supplemented with 0.3 mg/ml L-glutamine, 100 units/ml penicillin G potassium, 100 µg/ml streptomycin sulfate, and 40 µg/ml Gentacin (DMEM) and further supplemented with fetal bovine serum (JRH Biosciences) to be 10% was used in the culture of the cells. The cell fusion was performed by mixing the spleen cells of the immunized mouse with the Sp2/0-Ag14 cells, and adding thereto a polyethylene glycol solution (Sigma-Aldrich Co. LLC). The fused cells were cultured in HAT-RPMI [serum-supplemented RPMI containing 0.1 mM sodium hypoxanthine, 0.4 µM aminopterin and 0.1 mM thymidine (Gibco)], and antibody production in a culture supernatant was confirmed by an enzyme-linked immunosorbent assay (ELISA). Antibody production-positive cells were cultured for proliferation in HT-RPMI [serum-supplemented RPMI containing 0.1 mM sodium hypoxanthine and 0.1 mM thymidine].

Cloned cells were intraperitoneally inoculated to a mouse (BALB/c, retired, Japan SLC, Inc.) inoculated with 2,6,10,14-tetramethylpentadecane (Kishida Chemical Co., Ltd.), and ascitic fluid was collected. This ascitic fluid was applied to a protein G column to purify a monoclonal antibody. Finally, 83 clones of monoclonal antibody-producing cells were obtained against SARS-CoV-2NP.

### (Reference Example 3) Preparation of gold colloid suspension

Gold colloid particles were prepared in accordance with a routine method with an aqueous chloroauric acid solution (1 g of gold per L of aqueous solution; manufactured by Katayama Chemical Industries Co., Ltd.) as a raw material. Specifically, to 99 ml of pure water boiled by heating, 1 ml of a 1% (v/w) aqueous chloroauric acid solution was added, and further, 1.5 ml of a 1% (v/w) aqueous sodium citrate solution was added 1 minute later. The mixture was heated, boiled for 5 minutes, and then left at room temperature for cooling. Subsequently, this solution was adjusted to pH 9.0 by the addition of a 200 mM aqueous potassium carbonate solution, and the total amount was brought to 100 ml by the addition of ultrapure water thereto to obtain a gold colloid suspension containing gold colloid particles suspended therein. If the desired particle size was not obtained, gold colloid particles having a necessary particle size were prepared by appropriately adjusting, for example, the amount of the reducing agent relative to chloroauric acid.

### (Example 1) Separation of moiety that includes labeling substance (platinum-gold colloid particle) using pH adjuster

### (1) Preparation of platinum-gold colloid-labeled antibody solution

One anti-NP antibody selected from the anti-NP antibodies obtained in Reference Example 2 was labeled with platinum-gold colloid by the following procedures. 1 µg (protein-based weight) (hereinafter, the protein-based weight is indicated by the numerical value of a weight of the purified protein by weight analysis) of the anti-NP antibody and 1 mL of the platinum-gold colloid suspension having a particle size of 120 nm prepared in Reference Example 1 were mixed and left standing at room temperature for 2 minutes so that the anti-NP antibody was bound to the surfaces of the platinum-gold colloid particles. Then, a 10% aqueous bovine serum albumin (hereinafter, referred to as "BSA") solution was added thereto such that the final concentration was 1.0% based on the total amount of the liquid. Residual surfaces of the platinum-gold colloid particles were thus blocked with BSA to prepare a platinum-gold colloid-labeled anti-NP antibody solution. Then, this solution was centrifuged (600 × g, 25 min) to precipitate a platinum-gold colloid-labeled anti-NP antibody. The supernatant was removed to obtain a platinum-gold colloid-labeled anti-NP antibody. The obtained platinum-gold colloid-labeled anti-NP antibody was suspended in a 50 mM Tris-HCl buffer solution (pH 7.4) containing 10% saccharose, 1% BSA, and 0.5% Triton-X100 (trade name) to prepare a platinum-gold colloid-labeled antibody solution.

### (2) Cleavage treatment with pH adjuster

An antibody different from the anti-NP antibody used in platinum-gold colloid labeling among the anti-NP antibodies obtained in Reference Example 2 was adjusted to 20 µg/mL with a Tris-HCl buffer solution to prepare a diluted antibody solution. 100 µL of the diluted antibody solution was added to each well of a 96-well microplate, and the plate was left standing for 15 to 18 hours to immobilize the antibody on the solid phase. Then, blocking treatment was performed, and then, 50 µL of a SARS-CoV-2NP solution adjusted to 10 ng/mL and 50 µL of the platinum-gold colloid-labeled antibody solution were added thereto, and the plate was left standing at 37°C for 10 minutes. Subsequently, the plate was washed with TBS-T (Tris buffered saline with Tween 20), and the washing solution was discarded. Then, 100 µL each of various reagents differing in pH was added thereto, and the plate was left standing at room temperature for 2 minutes. Then, the solution in each well was recovered. The types and pH values of the reagents used are shown in Table 1. The pH values were measured using a pH meter (LAQUA manufactured by HORIBA Advanced Techno, Co., Ltd.).

The pH of the citric acid solutions was adjusted so as to obtain solutions having the desired pH (pH 2.94 to pH 6.96) by mixing citric acid with trisodium citrate. The phosphoric acid solutions were adjusted to the desired pH (pH 6.99 to 8.56) near neutrality by mixing sodium dihydrogen phosphate with disodium hydrogen phosphate, and adjusted to the desired alkaline pH (pH 11.33 to 12.85) by mixing disodium hydrogen phosphate with trisodium phosphate. The glycine hydrochloride buffer solutions, the glycine-NaOH buffer solutions, and the triethylamine solutions were adjusted to the desired pH by the addition of hydrochloric acid or sodium hydroxide. The pH of the hydrochloric acid solution and the sodium hydroxide solution was adjusted by changing a dilution ratio.

**Table 1**

| pH adjuster | pH value | | | | |
|---|---|---|---|---|---|
| Hydrochloric acid | 1.89 | | | | |
| Glycine-HCl | 1.53 | 2.48 | 3.18 | | |
| Citric acid | 2.94 | 3.97 | 4.92 | 5.93 | 6.96 |
| Phosphoric acid (neutral) | 6.99 | 7.97 | 8.56 | | |
| Glycine-NaOH | 8.60 | 8.98 | 10.51 | | |
| Triethylamine | 9.73 | 10.53 | 11.63 | | |
| Phosphoric acid (alkaline) | 11.33 | 12.09 | 12.85 | | |
| NaOH | 13.90 | | | | |

### (3) Measurement of platinum-gold colloid particle

The amount of the platinum-gold colloid particles contained was measured as to the solutions recovered by the operation described above. Specifically, the recovered solutions were introduced to sample cells, and the sample cells were irradiated with laser beam at 650 nm. The resulting scattered light was measured with photodiode set at a predetermined position.

Scattered light intensity is increased in proportion to the concentration of platinum-gold colloid particles in a solution. Therefore, the concentration of the platinum-gold colloid particles in the solution can be quantitatively analyzed by measuring the scattered light intensity. Specifically, the "moiety that includes the platinum-gold colloid particles" separated from the solid phase or the immune complex can be detected by treating the immune complex with the pH adjuster.

If the platinum-gold colloid-labeled anti-NP antibody is nonspecifically adsorbed (to a location other than the antigen), this antibody is not removed by a washing operation and is separated by the pH adjuster and mixed into a measurement sample. Accordingly, 100 µL of the platinum-gold colloid-labeled anti-NP antibody solution was added alone to each well treated in the same manner as above, and the plate was left standing at 37°C for 10 minutes and subsequently washed with TBS-T. Then, 100 µL of the pH adjuster solution was added thereto, and the plate was left standing at room temperature for 2 minutes, followed by the recovery of the solution from each well and scattered light measurement. The same operation as above was performed for each pH adjuster, and the obtained scattered light intensity was used as a blank value for each pH adjuster.

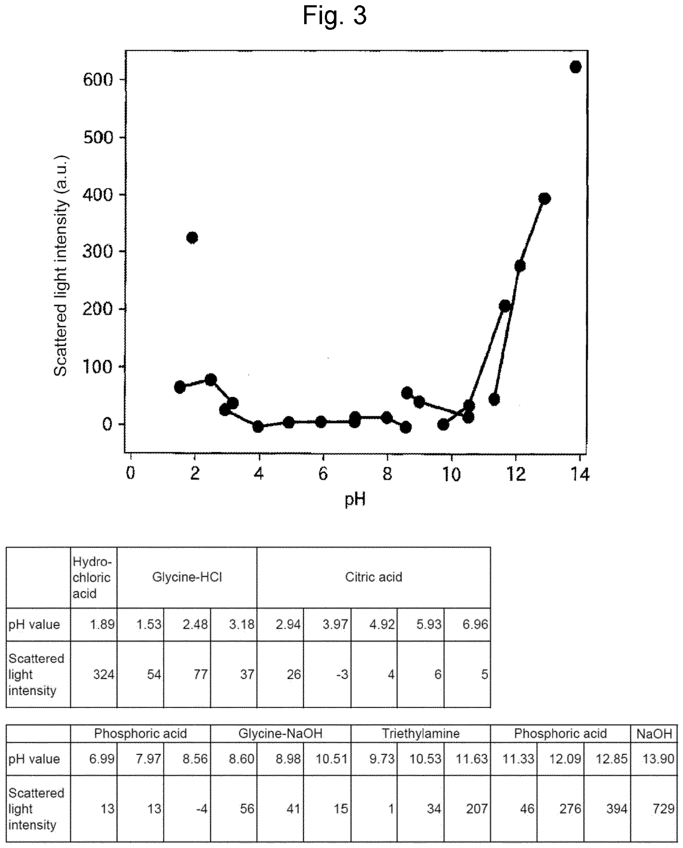

The blank value can be estimated as the scattered light intensity of the platinum-gold colloid particles derived from the platinum-gold colloid-labeled anti-NP antibody nonspecifically adsorbed in the well. Therefore, this blank value was subtracted from the measurement value, and the obtained value was regarded as the scattered light intensity of the "moiety that included the platinum-gold colloid particles" in the formed immune complex. The results are shown in Figure 3.

Figure 3 is a diagram showing the relationship between the pH value of each reagent and cleavage characteristics. The pH value of each reagent and the value of scattered light intensity are indicated in the lower part of Figure 3, and each value is shown in a graph in the upper part of Figure 3. The abscissa of the graph depicts the pH value, and the ordinate depicts the scattered light intensity. As also seen from Figure 3, the scattered light intensity was low when the pH value was around neutrality. In short, the added neutral reagents were found to have low cleavage performance of the "moiety that included the platinum-gold colloid particles" in the complex. On the other hand, the scattered light intensity was elevated when the added reagents were acidic or basic, indicating that the pH adjusters such as acids or bases were able to cleave the "moiety that included the platinum-gold colloid particles" in the complex bound to the solid phase (solid phase-anti-NP antibody-SARS-CoV-2NP-platinum-gold colloid-labeled anti-NP antibody).

Particularly, the pH adjusters of pH less than 4 (acids) and the pH adjusters of pH 10.6 or higher (bases) were found to have favorable cleavage performance, and further, the pH adjusters of pH 11.6 or higher (bases) were found to have excellent cleavage performance.

### (Example 2) Separation of moiety that includes labeling substance (platinum-gold colloid particle) using sodium hydroxide

As a result of Example 1, the cleaving ability of a sodium hydroxide solution was particularly favorable. Therefore, the cleavage performance of the moiety that included the labeling substance from the complex bound to the solid phase (solid phase-anti-NP antibody-SARS-CoV-2NP-platinum-gold colloid-labeled anti-NP antibody) was further evaluated by varying concentrations of sodium hydroxide.

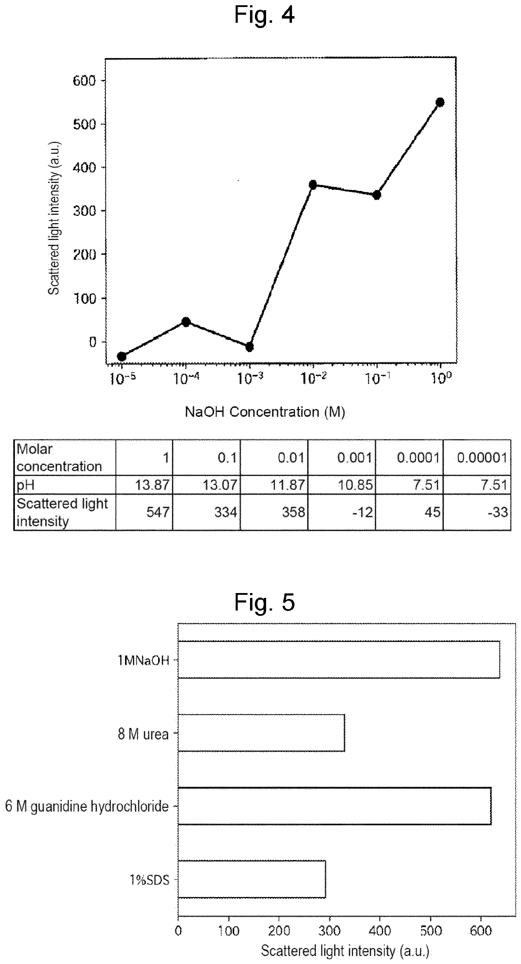

The sodium hydroxide concentrations were set to 0.01 mM, 0.1 mM, 1 mM, 10 mM, 100 mM, and 1 M. The same treatment as in Example 1 was performed except that 100 µL of an aqueous sodium hydroxide solution adjusted to each concentration was used. The results are shown in Figure 4.

Figure 4 is a diagram showing the cleavage performance of sodium hydroxide for the complex. The pH value and the value of scattered light intensity at each concentration of sodium hydroxide are indicated in the lower part of Figure 4, and each value is indicated in a graph in the upper part of Figure 4. The abscissa of the graph depicts the molar concentration of sodium hydroxide, and the ordinate depicts the scattered light intensity.

As also seen from Figure 4, sodium hydroxide having concentrations of 10 mM or higher was found to liberate the moiety that included the gold colloid particles from the immune complex. The 10 mM sodium hydroxide solution had pH of 11.87.

### (Example 3) Separation of moiety that includes labeling substance (platinum-gold colloid particle) using denaturant

A microplate having wells treated by the immobilization of the anti-NP antibody and blocking was prepared by the same method as in Example 1. The solution of each well was recovered in the same manner as in Example 1 except that a denaturant was allowed to act instead of the pH adjuster on the complex formed on the wells of this microplate as a solid phase (solid phase-anti-NP antibody-SARS-CoV-2NP-platinum-gold colloid-labeled anti-NP antibody). The amount of the platinum-gold colloid particles was measured in the same manner as in Example 1(3). 8 M urea, 6 M guanidine hydrochloride, or 1% SDS was used as the denaturant. The results are shown in Figure 5. For comparison, results of performing the same treatment as above using 1 M NaOH are also shown in Figure 5.

Figure 5 is a diagram showing the action of the denaturant on the immune complex bound to the solid phase in terms of the scattered light intensity of liberated gold colloid (moiety that includes the gold colloid particles). The case of using 1 M NaOH is indicated as comparative data. As also seen from Figure 5, all the denaturants were found to liberate the moiety that included the platinum-gold colloid particles.

### (Example 4) Separation of moiety that includes labeling substance (alkaline phosphatase)

The anti-NP antibody used in immobilization on the microplate in Example 1 was chemically bound to magnetic particles (manufactured by JSR Life Sciences, LLC) and thereby immobilized thereon. Also, 100 µg of the anti-NP antibody used in the platinum-gold colloid-labeled anti-NP antibody in Example 1 was labeled with alkaline phosphatase (ALP) using Alkaline Phosphatase Labeling kit (Dojindo Laboratories) to prepare an ALP-labeled anti-NP antibody solution. To 24 µL of a dispersion of 4 mg/mL anti-NP antibody-immobilized magnetic particles, 120 µL of the ALP-labeled antibody solution diluted 1000-fold with TBS-T was added, and 120 µL of a 1.2 or 12 ng/mL SARS-CoV-2NP solution was added, and the mixture was left standing at 37°C for 30 minutes. The magnetic particles were appropriately magnetically collected and washed with TBS-T. Then, 200 µL of a 100 mM glycine-sodium hydroxide buffer solution (pH 10.0) or a 100 mM triethylamine buffer solution (pH 11.5) was added thereto, and the mixture was stirred at room temperature for 2 minutes to liberate ALP (moiety that included the ALP label) into the solution. Subsequently, in the magnetically collected state of the magnetic particle, a supernatant (containing the liberated ALP) was collected and diluted 100-fold with TBS. To 5 µL of the resulting neutralized solution, 100 µL of a coloring reagent reaction test solution (thio NAD cycling reagent) given below was added, and the mixture was set in microplate reader SH-9000 (Corona Electric Co., Ltd.). Coloring reaction at 37°C was measured over time in terms of absorbance at 405 nm.

### Thio NAD cycling reagent

100 mM Tris buffer solution pH 9.8
1.8 mM thio NAD
0.9 mM NADH
3.8 mM MgCl2
25 units/ml 3α-hydroxysteroid dehydrogenase
5 mM androsterone 3-phosphate

As shown in Table 2, the coloring reaction at 405 nm was measured in terms of absorbance both by treatment with the glycine-sodium hydroxide buffer solution (pH 10.0) and by treatment with the triethylamine buffer solution (pH 11.5). Specifically, this treatment was found to be able to liberate the moiety that included the labeling substance ALP from the solid phase while maintaining the enzymatic activity of ALP.

**Table 2**

| 100 mM Glycine-NaOH (pH 10.0) | | 100 mM triethylamine (pH 11.5) | |
|---|---|---|---|
| Antigen concentration (ng/mL) | Absorbance [mAbs] | Antigen concentration (ng/mL) | Absorbance [mAbs] |
| 0 | 0 | 0 | 0 |
| 1.2 | -2 | 1.2 | 95 |
| 12.0 | 51 | 12.0 | 394 |

In this experiment, 1.2 ng/mL antigen was able to be detected at 95 mAbs (light path length = 3.03 mm). When the 100-fold dilution at the time of neutralization was taken into consideration, 12 pg/mL antigen was able to be detected. It is evident that 0.2 pM antigen molecule or alkaline phosphatase-labeled antibody was able to be detected when calculated from the molecular weight (49.7 kDa) of the antigen.

When one ALP molecule is present in a microwell having a volume of 1 pL, the ALP concentration is 1 pM. Provided that the shape of the microwell is 1 × 1 × 1000 µm, the light path length thereof is 1 mm and absorbance resulting from the reaction of one ALP molecule is presumed to exceed 100 mAbs (which means 166 mAbs at the light path length of 1 mm at 1 pM by linear estimation from the measurement value of the present Example), indicating being sufficiently detectable.

### (Example 5) Sensitization treatment of gold colloid particle

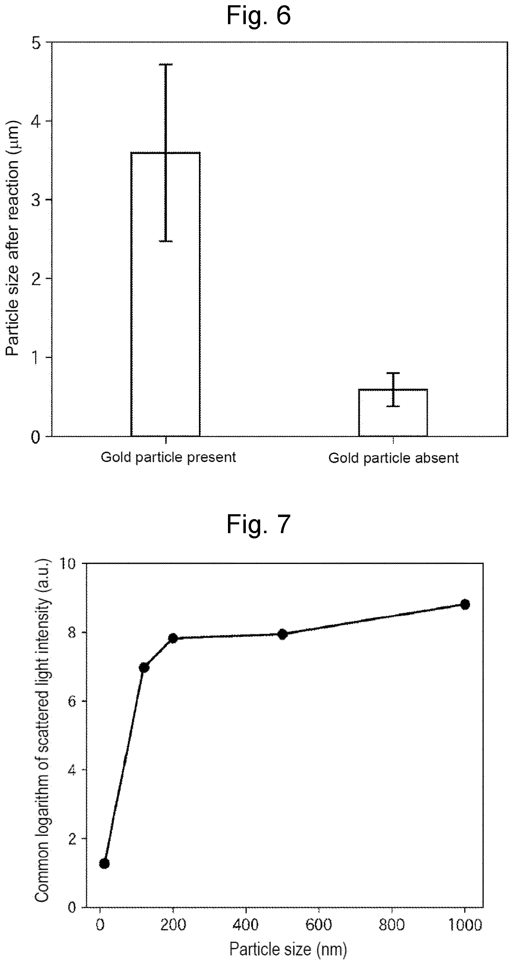

The gold colloid particle suspension (particle size: approximately 12 nm) prepared in Reference Example 3 was adjusted to a concentration of 4.36 nM with pure water, and a 2 µL aliquot thereof was added to 100 µL of 1 M NaOH and mixed. This treatment with 1 M NaOH was performed to simulate the cleavage treatment of the complex with the pH adjuster. 100 µL of 1 M HCl was added thereto for neutralization. 6.35 µL of this neutralized solution was added to a mixed solution of 972 µL of a 10 mM Walpole buffer (pH 2.0) and 1.65 µL of a 12.1 M chloroauric acid solution, and further, 20 µL of 375 mM hydroxylamine hydrochloride was added thereto and mixed. The mixture was left standing at room temperature for 10 minutes to perform sensitization reaction. Then, the gold colloid particles in the solution were sedimented by centrifugation, and the supernatant was removed, followed by washing with ultrapure water. 1 µL of the gold colloid particle dispersion thus obtained was added dropwise onto a sample holder to which a carbon tape was attached, then deaerated, and photographed under electron microscope TM4000 Plus (manufactured by Hitachi, Ltd.). The acceleration voltage was set to 15 kV, and the magnification was set to 1000. Five or more fields of view were photographed, and the images were analyzed to calculate an average particle size and a standard deviation. The same sensitization treatment as above was performed using ultrapure water free of gold colloid particles instead of the 4.36 nM gold colloid particle dispersion and used as a comparative subject. The results are shown in Figure 6.

Figure 6 is a diagram showing change in gold colloid particle size through sensitization reaction. The ordinate of Figure 6 depicts the particle size. As also seen from Figure 6, the gold colloid particles in the 4.36 nM gold colloid particle dispersion were able to grow into particles having a particle size of approximately 3 to 5 µm through the sensitization reaction. On the other hand, the precipitation of the gold colloid particles was also observed in the absence of the gold colloid particles, whereas the particles were markedly small as compared with the case of performing the sensitization treatment. Therefore, these particles were evidently distinguishable in the detection. Since the particle size is adjustable, as a matter of course, by adjusting reaction conditions, it is evident that a gold colloid particle having a particle size suitable for a detection system can be created.

This indicated that: in the case of forming an immune complex using a labeled antibody bound to a micro gold colloid particle, and then separating a moiety that includes the gold colloid particle (from the solid phase side), sensitization reaction can be performed for the gold colloid particle included in the separated moiety; and furthermore, the gold particle after the sensitization can be optically sufficiently detected.

### (Example 6) Change in scattered light intensity depending on particle size of metal particle

### (1) Preparation of gold colloid particles and platinum colloid particles having varying particle sizes

Platinum-gold colloid particles having a particle size of 120 nm were prepared by Reference Example 1. Gold colloid particles having particle sizes of 12 nm and 200 nm were prepared by Reference Example 3. Commercially available products (NANOPARTz, A11-500-CIT-DIH-1-50, A11-1000-NPC-DIH-1-100) were obtained as gold (colloid) particles having particle sizes of 500 nm and 1000 nm. The particles of each type were prepared in a state dispersed in a solution.

### (2) Measurement of scattered light intensity

The gold colloid particle dispersion having each particle size prepared as described above was diluted into a concentration in a range where scattered light was able to be measured. The measurement of scattered light was carried out by the same approach as in Example 1. The value of the obtained scattered light intensity was divided by the concentration to calculate scattered light intensity at 1 nM. As a result, the scattered light intensity was found to be increased with increase in the particle size of the gold colloid particles. The results are shown in Figure 7.

Figure 7 is a diagram showing the relationship between the particle size of the metal colloid particle and scattered light intensity. The abscissa depicts the particle size (nm), and the ordinate depicts the scattered light intensity in logarithmic expression. As also seen from Figure 7, the scattered light intensity was markedly increased from 120 nm, indicating that approximately 100 nm or larger metal colloid particles were detectable in a simple scattered light detection system of a laser light source and photodiode.

### (Example 7) Antigen detection based on moiety that includes labeling substance (platinum-gold colloid particle)

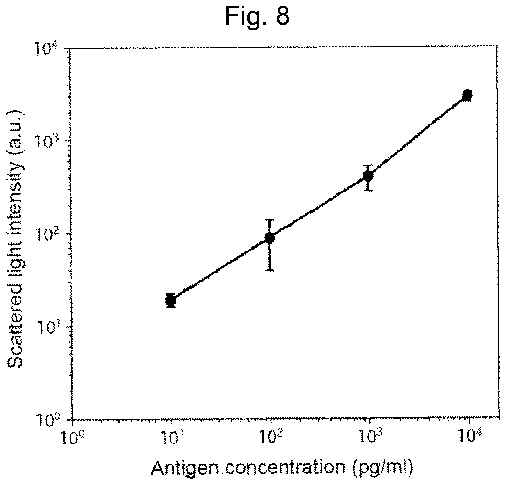

The anti-NP antibody used in immobilization on a solid phase in Example 1 was adjusted to 20 µg/mL with a Tris-HCl buffer solution to prepare a diluted antibody solution. This solution was mixed with magnetic particles (manufactured by JSR Life Sciences, LLC) so that the anti-NP antibody was immobilized on the magnetic particle through a chemical bond. Then, the anti-NP antibody immobilized on the magnetic particle, each SARS-CoV-2NP solution adjusted to a concentration of 10, 100, 1000, or 10000 pg/mL, and the platinum-gold colloid-labeled anti-NP antibody prepared in Example 1 were reacted at 37°C for 30 minutes. The magnetic particles were recovered by centrifugal filtration through a 0.45 µm centrifugal filter (Merck KGaA) from each solution after the reaction, and washed five times with TBS-T. Then, 1 M NaOH was added to the magnetic particles, and a complex bound to the magnetic particle (magnetic particle-anti-NP antibody-SARS-CoV-2NP-platinum-gold colloid-labeled anti-NP antibody) was subjected to cleavage treatment (elution). The scattered light intensity of the recovered eluate was measured. The measurement results are shown in Figure 8.

Figure 8 is a diagram showing the concentration of the eluted platinum-gold colloid particles (moiety that includes the platinum-gold colloid particles) in terms of scattered light intensity. The ordinate depicts the scattered light intensity in logarithmic expression, and the abscissa depicts the antigen concentration. As also seen from Figure 8, the scattered light intensity based on the platinum-gold colloid particles was in proportion to the antigen concentration. Specifically, as the antigen concentration was elevated, the amount of the eluted platinum-gold colloid particles was accordingly increased, demonstrating that: cleavage treatment was effectively carried out for the complex formed through antigen-antibody reaction on the solid phase; and furthermore, the cleaved moiety that included the labeling substance was separated, eluted, and appropriately detected.

### (Example 8) Separation of moiety that includes labeling substance (ALP) using competing agent

The same anti-NP antibody-immobilized magnetic particles as in Example 7 were adjusted to 4 mg/mL with TBS (Tris-buffered saline). To a 24 µL aliquot thereof, 120 µL of SARS-CoV-2NP was added at each concentration of 0, 0.1, 1, or 10 ng/mL to prepare a mixed solution. Then, to each mixed solution prepared, 120 µL of an ALP-labeled anti-NP antibody was added, and the mixture was stirred, reacted at 37°C for 10 minutes, and then dispensed at 110 µL/tube to 1.5 mL tubes. Then, while magnetically collected, the magnetic particles in the tube were washed five times with TBS-T. Then, 30 µL of an anti-NP antibody solution adjusted to 666 µg/mL with TBS-T was added thereto, and the mixture was reacted at room temperature for 10 minutes, followed by the recovery of a supernatant. The anti-NP antibody contained in the anti-NP antibody solution added here was the same antibody as the ALP-labeled anti-NP antibody added first except for the absence of the ALP label, and served as a competing agent against the ALP-labeled anti-NP antibody.

Subsequently, to the recovered supernatant, 100 µL of the same thio NAD cycling reagent as in Example 4 was added, and the mixture was set in microplate reader SH-9000 (Corona Electric Co., Ltd.). Coloring reaction at 37°C was measured over time in terms of absorbance at 405 nm. The absorbance value was obtained 60 minutes later and the absorbance value obtained similarly in antigen-free condition (blank) was subtracted to calculate a value. As a result, coloring reaction was confirmed with absorbance of 31 mAbs at 10 ng/mL SARS-CoV-2NP. Specifically, the moiety that included the ALP label was confirmed to be cleaved and eluted from the complex (magnetic particle-anti-NP antibody-SARS-CoV-2NP-ALS-labeled anti-NP antibody).

### Industrial Applicability

The present invention has applicability as a substance detection method and device in the medical field as well as various industrial fields because a target substance can be detected with great sensitivity and at a low cost.

### Reference Signs List

1: Disc
10: Sample reservoir
11: Sample addition port
12: Flow path
20: Trapping region
30: Branch tube
31, 32: Flow path
40: Waste liquid tank
50: Second reaction field
51: Outlet
52: Space
60a, 60b, 60c, 60d, 60e: Flow path
61, 62, 63, 64, 65: Liquid tank

## Claims

1. A target substance detection method comprising:
forming a complex in a first reaction field by sandwiching a target substance between a first trapping substance immobilized on a solid phase and a second trapping substance labeled with a labeling substance; separating a moiety that includes the labeling substance from the complex; moving the moiety to a second reaction field; and
detecting the target substance by a signal based on the labeling substance in the second reaction field.

2. The target substance detection method according to claim 1, wherein the solid phase is at least one selected from the group consisting of a surface, a plate-like body, a granular body, a fibrous body, a woven fabric, a nonwoven fabric, a film, and a sheet.

3. The target substance detection method according to claim 2, wherein the solid phase is a granular body and is at least one selected from the group consisting of a magnetic particle, a latex particle, and a resin particle.

4. The target substance detection method according to any one of claims 1 to 3, wherein the first trapping substance is at least one selected from the group consisting of an antibody, an antibody fragment, an engineered antibody, an aptamer and a nucleic acid that specifically binds to the target substance.

5. The target substance detection method according to any one of claims 1 to 4, wherein the second trapping substance is at least one selected from the group consisting of an antibody, an antibody fragment, an engineered antibody, an aptamer and a nucleic acid that specifically bind to the target substance.

6. The target substance detection method according to any one of claims 1 to 5, comprising removing the second trapping substance that fails to form the complex after formation of the complex.

7. The target substance detection method according to any one of claims 1 to 6, wherein the labeling substance is at least one selected from the group consisting of a metal colloid particle, an enzyme, a luminescent substance and a fluorescent substance.

8. The target substance detection method according to any one of claims 1 to 7, wherein the signal based on the labeling substance is generated in the second reaction field.

9. The target substance detection method according to claim 8, wherein the labeling substance is an enzyme, and the signal is generated by a reaction with a substrate in the second reaction field.

10. The target substance detection method according to claim 8, wherein the labeling substance is a metal colloid particle, and the signal is generated based on the metal colloid particle in the second reaction field.

11. The target substance detection method according to any one of claims 8 to 10, comprising carrying out an amplification reaction for amplifying the signal based on the labeling substance in the second reaction field.

12. The target substance detection method according to claim 11, wherein the labeling substance is a metal colloid particle, and the amplification reaction includes a reaction for allowing the metal colloid particle to grow by a reduction reaction with a metal ion as a substrate.

13. The target substance detection method according to any one of claims 1 to 12, wherein separating the moiety that includes the labeling substance from the complex is performed by at least one treatment selected from the group consisting of heating treatment, pH adjustment treatment, oxidation-reduction treatment, enzyme treatment and competitive reaction treatment.

14. The target substance detection method according to any one of claims 1 to 13, wherein the signal is at least one selected from the group consisting of transmitted light, reflected light, scattered light, luminescence and fluorescence.

15. The target substance detection method according to any one of claims 1 to 14, wherein the second reaction field has a volume smaller than a volume the first reaction field has.

16. The target substance detection method according to any one of claims 1 to 15, wherein the moiety that includes the labeling substance separated from the complex contains no solid phase.

17. A target substance detection device comprising at least:
a first reaction field where a complex is formed by sandwiching a target substance between a first trapping substance immobilized on a solid phase and a second trapping substance labeled with a labeling substance, and a moiety that includes the labeling substance is separated from the complex;
a second reaction field where the target substance is detected by a signal based on the labeling substance of the moiety separated from the complex in the first reaction field; and
a pathway where the moiety separated from the complex is moved from the first reaction field to the second reaction field.

18. A reagent for use in a target substance detection method, the method comprising:
forming a complex in a first reaction field by sandwiching a target substance between a first trapping substance immobilized on a solid phase and a second trapping substance labeled with a labeling substance;
separating a moiety that includes the labeling substance from the complex;
moving the moiety to a second reaction field; and
detecting the target substance by a signal based on the labeling substance in the second reaction field,
the reagent having an action of separating the moiety that includes the labeling substance.

19. The reagent according to claim 18, comprising at least one selected from the group consisting of a pH adjuster, a denaturant, a reducing agent, an enzyme, a competing agent, and an alcohol or ester and a derivative thereof in order to perform the action of separating the moiety that includes the labeling substance.

20. The reagent according to claim 19, comprising a pH adjuster in order to perform the action of separating the moiety that includes the labeling substance.
